# EUROPÄISCHE PATENTANMELDUNG

(11) **EP 4 759 795 A1**
(43) Veröffentlichungstag der Anmeldung: **17.06.2026**
(21) Anmeldenummer: 24218939.7
(22) Anmeldetag: 11.12.2024
(51) Int. Cl.: C07C 67/00, C07C 67/03, C07C 69/54, C08J 11/12

(54) **RECYCLE-MMA UND DESSEN VERWENDUNG FÜR DIE HERSTELLUNG HÖHERER ALKYLMETHACRYLATMONOMERE**

(71) Anmelder: Röhm GmbH, 64295 Darmstadt (DE)
(72) Erfinder: KRILL, Steffen, 64367 Mühltal (DE); TRESKOW, Marcel, 64293 Darmstadt (DE); HEIDEL, Sven, 64319 Pfungstadt (DE)
(74) Vertreter: Röhm Patent Association

(57) **Zusammenfassung**

Die vorliegende Erfindung betrifft ein neues Verfahren zur Isolierung von C₂-C₁₈-Alkyl(meth)acrylaten. In diesem Verfahren wird zunächst eine Polymerzusammensetzung, die mindestens ein Poly(alkyl(meth)acrylat) enthält, thermisch gespalten unter Erhalt mindestens eines Alkyl(meth)acrylats und mindestens eines weiteren Alkylesters. Diese Mischung wird anschließend kondensiert und mit einem Strom eines C₂-C₁₈-Alkyl(meth)acrylat-Herstellverfahrens gemischt. Durch Umsetzung mit einem C₂-C₁₈-Alkohol in Gegenwart eines Katalysators wird das C₂-C₁₈-Alkyl(meth)acrylat erhalten.

## Beschreibung

### Gebiet der Erfindung

Die vorliegende Erfindung betrifft ein neues Verfahren zur Isolierung von C₂-C₁₈-Alkyl(meth)acrylaten. In diesem Verfahren wird zunächst eine Polymerzusammensetzung, die mindestens ein Poly(alkyl(meth)acrylat) enthält, thermisch gespalten unter Erhalt mindestens eines Alkyl(meth)acrylats und mindestens eines weiteren Alkylesters. Diese Mischung wird anschließend kondensiert und mit einem Strom eines C₂-C₁₈-Alkyl(meth)acrylat-Herstellverfahrens gemischt. Durch Umsetzung mit einem C₂-C₁₈-Alkohol in Gegenwart eines Katalysators wird das C₂-C₁₈-Alkyl(meth)acrylat erhalten.

### Stand der Technik

Alkyl(meth)acrylate, insbesondere Methylmethacrylat (MMA), besitzen einen breiten Einsatzbereich.

MMA wird insbesondere zur Herstellung von Polymethylmethacrylat (PMMA) eingesetzt, welches sich durch hervorragende optische (Acrylglas) und andere physikalische Eigenschaften auszeichnet. Seine Einsatzgebiete umfassen Verglasungen und Fassaden in der Architektur, Beleuchtungselemente und Fahrzeugrückleuchten im Automobilbereich, Flugzeugfenster, Dekorations-, Design- oder Möbelelemente, Flachbildschirme und Displays, Lärmschutzwände und Lichtwerbungen.

Bei der Verarbeitung von PMMA fallen Produktionsabfälle, sogenannte Post Industrial-Abfälle, an. Darüber hinaus enthalten auch sogenannte Post Consumer-Abfälle, wie Elektro- und Altgeräteschrott, PMMA, häufig neben anderen Kunststoffen und Additiven. Der Anteil an MMA-Baueinheiten in den in den Abfällen enthaltenen PMMA-Polymeren ist unterschiedlich und kann von 75 % bis über 99 % liegen.

PMMA, sowohl als Post Industrial-Abfall, als auch als Post Consumer-Abfall, kann grundsätzlich in seine Monomere depolymerisiert und als sogenanntes Recycle-MMA wiederverwendet werden. Zur Depolymerisation sind im Stand der Technik verschiedene Verfahren, wie Depolymerisation in Metallbädern, Drehrohröfen, Wirbelschichtreaktoren oder Extrudern, beschrieben.

Beispielsweise beschreiben die Druckschriften DE 642289 C, US 2,030,901, DE 3146194 A1, EP 3 635 043 und US 2,470,361 die thermische Depolymerisation von PMMA, teilweise mit nachfolgender Aufreinigung der erhaltenen Monomere, beispielsweise mittels Destillation.

Thermisch-katalytische Depolymerisationen von PMMA mit anschließender Aufreinigung der erhaltenen Monomere sind beispielsweise in US 2,858,255, DE 2132716, WO 2019/003253, DE 19729065 und EP 2 895 576 beschrieben.

Ebenso bekannt ist die Depolymerisation von PMMA, teilweise zusammen mit anderen Polymeren, in einer Wirbelschicht. Dies ist beispielsweise in US 5,663,420, WO 2000/017149 und US 8,304,573 beschrieben.

US 3,494,958 beschreibt ein thermisches Depolymerisationsverfahren für PMMA. Das erhaltene Monomer kann anschließend mittels Destillation aufgereinigt werden. Die US 3,494,958 beschreibt, dass die Aufreinigung beispielsweise analog zur Aufreinigung in einem ACH-Prozess (C₃-Verfahren) erfolgen kann.

In den vorstehend beschriebenen Verfahren ist die Ausbeute an Monomeren teilweise nicht ausreichend. Zudem erzielen die im Stand der Technik beschriebenen Verfahren recycelte Alkyl(meth)acrylate, deren Reinheit nicht ausreichend ist zur Herstellung von Poly(alkyl(meth)acrylaten) ausreichender Qualität. Dies ist insbesondere der Fall, wenn in den Depolymerisationsprozessen Poly(methylmethacrylat) eingesetzt wird, das einen großen Anteil an Additiven, wie Schlagzähmodifizierern oder Pigmenten und/oder andere Polymeren enthält.

Verfahren zur Herstellung von höheren Alkyl(meth)acrylaten ausgehend von hochreinen niederen Alkyl(meth)acrylaten sind im Stand der Technik beschrieben.

DE 100 26 644 A1 beschreibt die Herstellung eines Esters einer ungesättigten Carbonsäure durch Umesterung mit einem C₄-C₂₀-Alkohol in Gegenwart eines Umesterungskatalysators, der einen 2,2,6,6-Tetraalkyl-1-oxyl-piperidin-4-Rest enthält.

Die DE 102 00 171 A1 beschreibt ein Verfahren zur kontinuierlichen Herstellung von höheren (Meth)acrylsäureestern durch Umsetzung eines Methyl(meth)acrylats mit einem C₂-C₁₂-Alkohol. Die Aufreinigung des Produktes erfolgt mittels Destillation und anschließender Überführung des erhaltenen Destillationssumpfs in einen Verdampfer.

Die EP 1 583 733 B1 beschreibt ein Verfahren zur kontinuierlichen Herstellung von höheren (Meth)acrylsäureestern durch Umesterung von (Meth)acrylsäuremethylestern mit höheren Alkoholen in Gegenwart eines Katalysators.

Die im Stand der Technik beschriebenen Verfahren zur Herstellung von höheren Alkyl(meth)acrylaten gehen jeweils von hochreinen niederen Alkyl(meth)acrylaten aus. Keines der beschriebenen Verfahren kann mit den besonderen Anforderungen, die die Verwendung von depolymerisiertem PMMA (Recycle-MMA) mit reduzierter Reinheit mitbringt, umgehen. Dies macht die Verfahren teuer und wenig nachhaltig.

### Aufgabe

Es bestand daher Bedarf ein verbessertes Verfahren zur Isolierung von C₂-C₁₈-Alkyl(meth)acrylaten bereitzustellen, das die Nachteile der im Stand der Technik beschriebenen Verfahren nicht oder nur in vermindertem Maße aufweist. Insbesondere soll das Verfahren einen geringeren Ausstoß an klimawirksamen Abfallstoffen wie CO₂ aufweisen.

### Lösung

Gelöst wurde diese Aufgabe durch ein Verfahren zur Isolierung von C₂-C₁₈-Alkyl(meth)acrylaten umfassend die folgenden Schritte a) bis e):
a) thermische Spaltung von mindestens einer Polymerzusammensetzung, die mindestens ein Poly(alkyl(meth)acrylat) enthält, unter Erhalt eines ersten Gasstroms, der mindestens ein Alkyl(meth)acrylat und mindestens einen weiteren Alkylester enthält,
b) Kondensation des in Schritt a) erhaltenen ersten Gasstroms unter Erhalt eines flüssigen ersten Stroms, der das mindestens eine Alkyl(meth)acrylat und den mindestens einen weiteren Alkylester enthält,
c) Mischen des in Schritt b) erhaltenen flüssigen ersten Stroms mit einem weiteren Strom, der mindestens ein weiteres Alkyl(meth)acrylat enthält, wobei der weitere Strom Teil eines C₂-C₁₈-Alkyl(meth)acrylat-Herstellverfahrens ist, unter Erhalt eines Mischstroms, der den flüssigen ersten Strom und den weiteren Strom umfasst,
d) Umsetzen des in Schritt c) erhaltenen Mischstroms mit einem C₂-C₁₈-Alkohol in Gegenwart eines Katalysators unter Erhalt eines Produktstroms, der das mindestens eine Alkyl(meth)acrylat, das mindestens eine weitere Alkyl(meth)acrylat, das C₂-C₁₈-Alkyl(meth)acrylat, den Katalysator, den mindestens einen Alkylester, den C₂-C₁₈-Alkohol und mindestens einen weiteren Alkohol enthält,
e) Abtrennung des C₂-C₁₈-Alkyl(meth)acrylats aus dem Produktstrom.

Es wurde überraschend gefunden, dass durch das erfindungsgemäße Verfahren C₂-C₁₈-Alkyl(meth)acrylate mit einer Reinheit von > 99,4% erhalten werden.

Insbesondere können durch das erfindungsgemäße Verfahren überraschenderweise Nebenprodukte wie weitere Alkylester, sowie deren Stoffgemische und Azeotrope effizient entfernt werden, selbst wenn sich deren Normalsiedetemperatur nur geringfügig, beispielsweise um +/- 1 K (Kelvin), vorzugsweise +/- 0,6 K von der Normalsiedetemperatur des Alkyl(meth)acrylats und/oder dessen Azeotropen unterscheidet. Dies ist insbesondere durch Verfahrensschritte d) und e) bedingt.

Auch Additive und Kettenregler, die in der Polymerzusammensetzung enthalten sein können, und die aus ihnen bei der thermischen Spaltung der in der Polymerzusammensetzung erhaltenen Produkte wie Mercaptane lassen sich einfach und kostengünstig durch das erfindungsgemäße Verfahren abtrennen. Beispielsweise enthalten die im erfindungsgemäßen Verfahren erhaltenen C₂-C₁₈-Alkyl(meth)acrylate maximal 0,6 % Nebenprodukte, und davon maximal 0,5 % kritische ungesättigte Nebenprodukte. Aufgrund des erfindungsgemäßen Verfahrens und der dadurch möglichen effizienten Abtrennung der Nebenprodukte, weisen die erfindungsgemäß hergestellten C₂-C₁₈-Alkyl(meth)acrylate trotz der in der Polymerzusammensetzung enthaltenen Mercaptane keinen oder nur einen sehr geringen Geruch auf.

Das erfindungsgemäße Verfahren und mit dem erfindungsgemäßen Verfahren hergestellte C₂-C₁₈-Alkyl(meth)acrylate weisen zudem einen geringeren COz-Ausstoß und damit einen besonders niedrigen Carbon Footprint auf. Dies wird insbesondere dadurch erzielt, dass das erfindungsgemäße Verfahren auch die Verwendung von Polymerzusammensetzungen mit einem relativ hohen Anteil an Additiven und sonstigen Verunreinigungen erlaubt und dass die Rohstoffquelle wiederverwendbar ist.

Das erfindungsgemäße Verfahren ist somit den bisher bekannten überlegen, da es der Anforderung gerecht wird, sich selbst in der Kreislaufkette wiederverwenden zu können (circular economy).

Zudem können die bei der Depolymerisation erhaltenen Nebenprodukte nach ihrer Abtrennung weiterverwertet werden, sodass der Carbon Footprint und damit das global warming potential (GWP) weiter reduziert wird.

Nachfolgend wird das erfindungsgemäße Verfahren näher beschrieben.

In Schritt a) des erfindungsgemäßen Verfahrens wird mindestens eine Polymerzusammensetzung thermisch gespalten unter Erhalt eines ersten Gasstroms. Die mindestens eine Polymerzusammensetzung enthält mindestens ein Poly(alkyl(meth)acrylat). Der Gasstrom enthält mindestens ein Alkyl(meth)acrylat und mindestens einen weiteren Alkylester.

Der Begriff "mindestens eine Polymerzusammensetzung" bedeutet im Rahmen der vorliegenden Erfindung sowohl genau eine Polymerzusammensetzung als auch eine Mischung aus zwei oder mehreren Polymerzusammensetzungen. Eine Mischung aus zwei oder mehreren Polymerzusammensetzungen ist erfindungsgemäß bevorzugt.

Die mindestens eine Polymerzusammensetzung enthält mindestens ein Poly(alkyl(meth)acrylat).

"Mindestens ein Poly(alkyl(meth)acrylat)" bedeutet im Rahmen der vorliegenden Erfindung sowohl genau ein Poly(alkyl(meth)acrylat) als auch eine Mischung aus zwei oder mehreren Poly(alkyl(meth)acrylaten). Unter "Poly(alkyl(meth)acrylaten)" werden im Rahmen der vorliegenden Erfindung Polymere und Copolymere von Alkyl(meth)acrylaten verstanden.

Copolymere von Alkyl(meth)acrylaten sind beispielsweise Copolymere von Alkyl(meth)acrylaten mit 1-Alkenen, anderen Alkyl(meth)acrylaten, (Meth)acrylsäure, Styrol, Polyestern und/oder Polyurethan(meth)acrylaten.

1-Alkene, die mit Alkyl(meth)acrylaten copolymerisieren können, sind als solche bekannt und beispielsweise 1-Hexen, 1-Hepten, Vinylcyclohexan, 3,3-Dimethyl-1-propen, 3-Methyl-1-diisobutylen und 4-Methylpenten-1.

Unter dem Begriff "Styrol" wird im Rahmen der vorliegenden Erfindung nicht nur Styrol als solches verstanden, sondern auch substituierte Styrole wie beispielsweise α-Methylstyrol, α-Ethylstyrol, Vinyltoluol, p-Methylstyrol, Monochlorstyrole, Dichlorstyrole und Tribromstyrole.

Geeignete Polyester sind als solche bekannt und vorzugsweise via Polykondensation oder ringöffnende Polymerisation erhältlich.

Unter "Polyurethan(meth)acrylaten" werden im Rahmen der vorliegenden Erfindung (Meth)acrylate verstanden, die über Urethangruppen miteinander verknüpft sind. Sie sind durch Umsetzung von Hydroxyalkyl(meth)acrylaten mit Polyisocyanaten und Polyoxyalkylenen, die mindestens zwei Hydroxyfunktionalitäten aufweisen, erhältlich. Anstelle von Hydroxyalkyl(meth)acrylaten können auch Ester der (Meth)acrylsäure mit Oxiranen, wie beispielsweise Ethylenoxid oder Propylenoxid, oder entsprechende Oligooxirane bzw. Polyoxirane verwendet werden. Geeignete Polyurethan(meth)acrylate sind als solche bekannt.

Die mindestens eine Polymerzusammensetzung kann beispielsweise aus Produktionsabfällen stammen. Dann handelt es sich bei der Polymerzusammensetzung üblicherweise um sogenannte Post Industrial-Abfälle, wie beispielsweise Angüsse, Anfahrklumpen bei der Extrusion, Stäube, Späne, anpolymerisierte Polymersirupe, Randabschnitte, Verschnitte bei Platten, Ausschuss bei Platten, Folien, Blöcke, Halbzeuge, Fehlfabrikationen von Formteilen oder Abfälle beim Spritzgießen.

Ebenso ist es möglich, dass die mindestens eine Polymerzusammensetzung aus sogenannten Post Consumer-Abfällen stammt. Dann handelt es sich üblicherweise um Abfälle von beispielsweise Elektro- und Altgeräteschrott, Gewächshäuser, Messebauten, Shop-Fittinge oder Lichtwerbung.

Die mindestens eine Polymerzusammensetzung enthält daher üblicherweise zumindest eine weitere Komponente. Die zumindest eine weitere Komponente ist beispielsweise ausgewählt aus der Gruppe bestehend aus von dem Poly(alkyl(meth)acrylat) verschiedenen Polymeren, Pigmenten, Farbstoffen, Füllstoffen, Hilfsstoffen, Reglern, Initiatoren, Schlagzähmachern, Trennmitteln und UV-Additiven.

Bevorzugt ist daher auch ein Verfahren, bei dem die Polymerzusammensetzung in Schritt a) zumindest eine weitere Komponente, ausgewählt aus der Gruppe bestehend aus von Poly(alkyl(meth)acrylat) verschiedenen Polymeren, Pigmenten, Farbstoffen, Füllstoffen, Hilfsstoffen, Reglern, Initiatoren, Schlagzähmachern, Trennmitteln und UV-Additiven, enthält.

Als von Poly(alkyl(meth)acrylat) verschiedene Polymere kommen insbesondere solche Polymere in Betracht, die üblicherweise als Blend mit Poly(alkyl(meth)acrylat) eingesetzt werden können. Hierzu zählen beispielsweise Polyethylen, Polyvinylchlorid, Polystyrol, Polyamide und Biopolymere wie α-Polysaccharide (Stärke), β-Polysaccharide (Cellulose, Chitin) Lignin und Polylactid.

Pigmente sind beispielsweise weiße, rote, blaue, grüne und/oder gelbe anorganische Pigmente. Besonders bevorzugt sind weiße, anorganische Pigmente wie Titandioxid.

Farbstoffe sind beispielsweise dem Fachmann bekannte organische Farbstoffe.

Typische Füllstoffe sind insbesondere mineralische Füllstoffe. Mineralische Füllstoffe sind vorzugsweise ausgewählt aus der Gruppe bestehend aus Calciumcarbonat, Bariumsulfat, Quarz, Quarzmehl, gefällten Kieselsäuren, pyrogenen Kieselsäuren, Korunde, Glasperlen und Cristobaliten.

Typische Hilfsstoffe sind als solche bekannt und beispielsweise ausgewählt aus der Gruppe bestehend aus Weichmachern, Paraffinen und/oder Inhibitoren.

Als Weichmacher werden vorzugsweise Ester, Polyole, Öle, niedermolekulare Polyether oder Phthalate eingesetzt.

Paraffine, die in der Polymerzusammensetzung enthalten sein können, sind als solche bekannt. Es können beispielsweise mehrere Paraffine mit unterschiedlichen Schmelzpunkten in unterschiedlichen Konzentrationen enthalten sein.

Aus der Gruppe der Inhibitoren sind vorzugsweise substituierte Phenole, Hydrochinonderivate, Phosphine und/oder Phosphite enthalten.

Als Regler kommen insbesondere aus der radikalischen Polymerisation bekannte Verbindungen, die die Kettenlängen regeln, in Betracht. Üblicherweise umfassen Kettenregler Mercaptane wie n-Dodecylmercaptan, aber auch mehrwertige Mercaptoverbindungen wie Pentaerythroltetrathioglycolat.

Initiatoren sind als solche ebenfalls bekannt und beispielsweise ausgewählt aus der Gruppe bestehend aus Peroxiden, Azoverbindungen, Persulfaten und Mischungen daraus.

Schlagzähmacher sind als solche bekannt und beispielsweise Polymerpartikel, die ein Elastomer enthalten.

Als Trennmittel kommen insbesondere langkettige Wachssäuren, wie Stearinsäure, Palmitinsäure oder Laurinsäure, sowie einwertige Fett- oder Wachsalkohole, wie beispielsweise Diethylenglycolmonopropylether, in Betracht.

Als UV-Additive kommen insbesondere UV-Stabilisatoren in Frage. Vorzugsweise werden die UV-Stabilisatoren ausgewählt aus der Gruppe bestehend aus Benzophenonderivaten, Benzotriazolderivaten, Thioxanthonatderivaten, Piperidinolcarbonsäureesterderivaten und Zimtsäureesterderivaten.

Die thermische Spaltung der mindestens einen Polymerzusammensetzung kann nach dem Fachmann bekannten Methoden erfolgen.

Die thermische Spaltung kann in dem Fachmann bekannten Reaktoren für thermische Spaltungen durchgeführt werden. Beispielsweise kann die thermische Spaltung in einem Pyrolysereaktor, in einem Extruder, in einem Drehrohrofen, einer Wirbelschichtpyrolyse, in einem Metallbad und/oder als trockene Destillation durchgeführt werden.

Die Polymerzusammensetzung kann bei der thermischen Spaltung in fester oder flüssiger Form vorliegen. Liegt die Polymerzusammensetzung in fester Form vor, so kann sie als reiner Feststoff vorliegen. Ebenso ist es möglich, dass die Polymerzusammensetzung in fester Form in einem Medium dispergiert vorliegt. Das Medium, in dem die Polymerzusammensetzung dispergiert vorliegen kann, kann beispielsweise ein Feststoff, wie Quarz, Metallspäne oder Kieselgur, sein. Ebenso ist es möglich, dass das Medium ein Gas ist wie beispielsweise Stickstoff oder eine Flüssigkeit, wie beispielsweise Wasser oder ein Kohlenwasserstoff. Es ist möglich, dass das Medium bei Raumtemperatur eine Flüssigkeit ist, bei den Bedingungen der thermischen Spaltung in Schritt a) allerdings als Gas vorliegt.

Die Polymerzusammensetzung liegt beispielsweise in flüssiger (geschmolzener) Form vor, wenn die thermische Spaltung in einem Extruder erfolgt.

Die Polymerzusammensetzung kann, insbesondere wenn sie in fester Form vorliegt, vor der thermischen Spaltung mechanisch zerkleinert werden, beispielsweise auf eine durchschnittliche Teilchengrößenverteilung von unter 6 mm (Qr, d50) bevorzugt unter 1,5 mm (Qr, d50), beispielsweise auf eine Teilchengrößenverteilung im Bereich von 0,1 mm (Qr, d50) bis 6 mm (Qr, d50). Vorzugsweise wird die Teilchengrößenverteilung mittels Laserdiffraktometrie bestimmt.

Die Temperatur (T) bei der thermischen Spaltung in Schritt a) liegt beispielsweise in einem Bereich von 240 °C bis 800 °C, bevorzugt bei einer Temperatur im Bereich von 300 °C bis 500 °C, besonders bevorzugt bei einer Temperatur im Bereich von 325 °C bis 400 °C.

Der Druck bei der thermischen Spaltung in Schritt a) liegt beispielsweise im Bereich von 200 mbar bis 1000 bar, bevorzugt im Bereich von 400 mbar bis 500 bar.

Erfindungsgemäß bevorzugt ist daher auch ein Verfahren, bei dem die Temperatur bei der thermischen Spaltung in Schritt a) im Bereich von 240 °C bis 800 °C liegt und/oder der Druck bei der thermischen Spaltung in Schritt a) im Bereich von 400 mbar bis 500 bar liegt.

Die thermische Spaltung in Schritt a) kann in Gegenwart eines Katalysators erfolgen. Für die thermische Spaltung geeignete Katalysatoren sind als solche bekannt und beispielsweise ausgewählt aus der Gruppe bestehend aus geschmolzenem Metall, Peroxiden, Kaliumsalzen wie beispielsweise Kaliumacetat, und Alkalimetallpersulfaten, wie beispielsweise Kaliummonopersulfat. Als geschmolzenes Metall eignen sich insbesondere Blei und/oder eutektische Blei/Zinn Mischungen.

Erfolgt die thermische Spaltung in Schritt a) in Gegenwart eines Katalysators, so wird die Spaltung in Schritt a) auch als thermisch katalytische Spaltung bezeichnet.

Erfindungsgemäß bevorzugt ist daher auch ein Verfahren, bei dem die thermische Spaltung in Schritt a) eine thermisch katalytische Spaltung ist.

Bei der thermischen Spaltung in Schritt a) wird das Poly(alkyl(meth)acrylat) gespalten. Diese Spaltung wird auch als Depolymerisation bezeichnet. Depolymerisationen als solche sind dem Fachmann bekannt. Bei Depolymerisationen wird ein Polymer in seine Monomer- und Oligomereinheiten gespalten.

Bei der thermischen Spaltung in Schritt a) wird also das Poly(alkyl(meth)acrylat) in seine Monomer- und Oligomereinheiten gespalten. Wie vorstehend ausgeführt, ist das Poly(alkyl(meth)acrylat) ein Polymer oder Copolymer von mindestens einem Alkyl(meth)acrylat. Bei der thermischen Spaltung bildet sich daher mindestens ein Alkyl(meth)acrylat.

Bei der thermischen Spaltung der Polymerzusammensetzung bildet sich zudem mindestens ein weiterer Alkylester. "Mindestens ein weiterer Alkylester" bedeutet im Rahmen der vorliegenden Erfindung sowohl genau ein weiterer Alkylester als auch eine Mischung aus zwei oder mehreren weiteren Alkylestern. Eine Mischung aus zwei oder mehreren weiteren Alkylestern ist erfindungsgemäß bevorzugt.

Der erste Gasstrom enthält daher das bei der thermischen Spaltung gebildeten mindestens eine Alkyl(meth)acrylat und den mindestens einen weiteren Alkylester.

Das mindestens eine Alkyl(meth)acrylat ist, wie vorstehend ausgeführt, von dem Poly(alkyl(meth)acrylat) abgeleitet.

"Mindestens ein Alkyl(meth)acrylat" bedeutet im Rahmen der vorliegenden Erfindung sowohl genau ein Alkyl(meth)acrylat als auch eine Mischung aus zwei oder mehreren Alkyl(meth)acrylaten. Eine Mischung aus zwei oder mehreren Alkyl(meth)acrylaten ist bevorzugt.

Unter "Alkyl(meth)acrylaten" werden im Rahmen der vorliegenden Erfindung Alkylester von (Meth)acrylsäure verstanden, die 1 bis 18, bevorzugt 1 bis 12, insbesondere bevorzugt 1 bis 4, Kohlenstoffatome im Alkylrest aufweisen. Der Alkylrest kann linear, zyklisch und/oder verzweigt sein. Darüber hinaus kann er aromatische Reste aufweisen. Der Alkylrest kann zudem Heteroatome innerhalb des Alkylrests aufweisen und/oder mit Heteroatomen substituiert sein, wie dies beispielsweise bei Hydroxypropyl(meth)acrylat und/oder Hydroxyethyl(meth)acrylat der Fall ist. Beispielsweise sind erfindungsgemäße Alkyl(meth)acrylate ausgewählt aus der Gruppe bestehend aus Methyl(meth)acrylat, Ethyl(meth)acrylat, 1-Methyl-Ethyl(meth)acrylat, Propyl(meth)acrylat, n-Butyl(meth)acrylat, Isobutyl(meth)acrylat, 2-Ethylhexyl(meth)acrylat, Isopentyl(meth)acrylat, Cyclohexyl(meth)acrylat, Tetrahydrofurfuryl(meth)acrylat, Allyl(meth)acrylat, Polyethylenglycol(meth)acrylat, Stearyl(meth)acrylat, Benzyl(meth)acrylat, Vinyl(meth)acrylat, Hydroxypropyl(meth)acrylat, Hydroxyethyl(meth)acrylat und Lauryl(meth)acrylat.

In einer weiteren Ausführungsform der vorliegenden Erfindung werden unter "Alkyl(meth)acrylaten" auch Polyethylenglycol(meth)acrylate mit einem gewichtsmittleren Molekulargewicht Mw im Bereich von 250 g/mol bis 10.000 g/mol verstanden.

Der Begriff "(Meth)acrylsäure" umfasst im Rahmen der vorliegenden Erfindung sowohl Acrylsäure als auch Methacrylsäure. Der Begriff "(Meth)acrylate" umfasst im Rahmen der vorliegenden Erfindung sowohl Acrylate als auch Methacrylate.

Unter "Alkyl(meth)acrylaten" werden im Rahmen der vorliegenden Erfindung daher sowohl Alkylmethacrylate als auch Alkylacrylate verstanden. Alkylmethacrylate sind erfindungsgemäß bevorzugt. Das mindestens eine Alkyl(meth)acrylat ist beispielsweise ein C₁-C₁₈-Alkyl(meth)acrylat, bevorzugt ein C₁-C₁₂-Alkyl(meth)acrylat und besonders bevorzugt ein C₁-C₄-Alkyl(meth)acrylat. Erfindungsgemäß am meisten bevorzugt umfasst das mindestens eine Alkyl(meth)acrylat Methyl(meth)acrylat.

Bevorzugt ist daher auch ein Verfahren, bei dem das in dem ersten Gasstrom in Schritt a) enthaltene mindestens eine Alkyl(meth)acrylat ausgewählt ist aus der Gruppe bestehend aus C₁- bis C₄-Alkyl(meth)acrylaten.

Das mindestens eine Alkyl(meth)acrylat weist beispielsweise eine Siedetemperatur bei Normaldruck im Bereich von 50 °C bis 300 °C, bevorzugt im Bereich von 80 °C bis 250 °C, auf.

Es versteht sich von selbst, dass der mindestens eine weitere Alkylester von dem mindestens einen Alkyl(meth)acrylat verschieden ist. Der mindestens eine weitere Alkylester umfasst vorzugsweise keine (Meth)acrylat-Einheit.

Beispielsweise ist der mindestens eine weitere Alkylester ausgewählt aus der Gruppe bestehend aus C₁-C₄-Alkylisobutyraten, C₁-C₄-Alkylpropionaten, C₁-C₄-Alkylpivalaten und Dicarbonsäurediestern.

Unter C₁-C₄-Alkylisobutyraten werden im Rahmen der vorliegenden Erfindung Alkylester der Isobuttersäure verstanden, die 1 bis 4 Kohlenstoffatome im Alkylrest aufweisen. Der Alkylrest kann linear oder verzweigt sein. Er kann zudem Heteroatome innerhalb des Alkylrests aufweisen und/oder mit Heteroatomen substituiert sein. Ein C₁-C₄-Alkylisobutyrat, das Heteroatome innerhalb des Alkylrests aufweist, ist beispielsweise Methyl-2-methoxyisobutyrat und Methyl-3-methoxyisobutyrat. Erfindungsgemäße C₁-C₄-Alkylisobutyrate sind beispielsweise ausgewählt aus der Gruppe bestehend aus Methylisobutyrat, Ethylisobutyrat, Methyl-2-methoxyisobutyrat und Methyl-3-methoxyisobutyrat.

Unter C₁-C₄-Alkylpropionaten werden im Rahmen der vorliegenden Erfindung Alkylester der Propionsäure verstanden, die 1 bis 4 Kohlenstoffatome im Alkylrest aufweisen. Der Alkylrest kann linear oder verzweigt sein. Er kann zudem Heteroatome innerhalb des Alkylrests aufweisen und/oder mit Heteroatomen substituiert sein. Erfindungsgemäße C₁-C₄-Alkylpropionate sind beispielsweise ausgewählt aus der Gruppe bestehend aus Methylpropionat und Ethylpropionat.

Unter C₁-C₄-Alkylpivalaten werden im Rahmen der vorliegenden Erfindung Alkylester der Pivalinsäure verstanden, die 1 bis 4 Kohlenstoffatome im Alkylrest aufweisen. Der Alkylrest kann linear oder verzweigt sein. Er kann zudem Heteroatome innerhalb des Alkylrests aufweisen und/oder mit Heteroatomen substituiert sein. Erfindungsgemäße C₁-C₄-Alkylpivalate sind beispielsweise ausgewählt aus der Gruppe bestehend aus Methylpivalat und Ethylpivalat.

Unter Dicarbonsäurediestern werden im Rahmen der vorliegenden Erfindung Alkylester von Dicarbonsäuren verstanden, die 1 bis 4 Kohlenstoffe im Alkylrest aufweisen. Ebenso werden unter Dicarbonsäurediestern Carbonsäureester von Diolen verstanden, bei denen beide Hydroxygruppen mit einer Carbonsäure verestert sind. Erfindungsgemäße Dicarbonsäurediester sind beispielsweise ausgewählt aus der Gruppe bestehend aus Propandisäure-dimethylester, Pentandisäuredimethylester, Hexandisäure-dimethylester und Heptandisäure-dimethylester.

Vorzugsweise ist der mindestens eine weitere Alkylester ausgewählt aus der Gruppe bestehend aus Methylpropionat, Ethylpropionat, Methylisobutyrat, Methylpivalat, Methyl-3-methoxyisobutyrat und Dicarbonsäurediestern.

Bevorzugt ist daher auch ein Verfahren, bei dem der mindestens eine weitere Alkylester ausgewählt ist aus der Gruppe bestehend aus Methylpropionat, Methylisobutyrat, Methylpivalat, Methyl-3-methoxyisobutyrat und Dicarbonsäurediestern.

Der mindestens eine weitere Alkylester weist beispielsweise eine Siedetemperatur bei Normaldruck im Bereich von 50 °C bis 200 °C, bevorzugt im Bereich von 75 °C bis 150 °C, auf.

Erfindungsgemäß weiterhin bevorzugt unterscheidet sich der Siedepunkt bei Normaldruck von zumindest einem mindestens einem weiteren Alkylester von dem Siedepunkt bei Normaldruck von zumindest einem mindestens einem Alkyl(meth)acrylat um im Bereich von -20 °C bis +20 °C, bevorzugt um im Bereich von -1 °C bis +1 °C, besonders bevorzugt um im Bereich von -0,6 °C bis +0,6 °C.

Ebenso bevorzugt unterscheidet sich der Siedepunkt eines Azeotrops aus zumindest einem mindestens einem weiteren Alkylester mit dem C₂-C₁₈-Alkohol und/oder dem mindestens einen weiteren Alkohol von dem Siedepunkt eines Azeotrops aus zumindest einem Alkyl(meth)acrylat mit dem C₂-C₁₈-Alkohol und/oder dem mindestens einen weiteren Alkohol um im Bereich von -20 °C bis +20 °C, bevorzugt um im Bereich von -1 °C bis +1 °C, besonders bevorzugt um im Bereich von - 0,6 °C bis +0,6 °C, wobei der Siedepunkt jeweils auf den Siedepunkt bei Normaldruck bezogen ist.

Wie vorstehend ausgeführt, wird der erste Gasstrom durch thermische Spaltung der mindestens einen Polymerzusammensetzung erhalten. Üblicherweise enthält der erste Gasstrom daher mindestens eine weitere Komponente. Es versteht sich von selbst, dass die mindestens eine weitere Komponente von dem mindestens einen Alkyl(meth)acrylat und dem mindestens einen weiteren Alkylester verschieden ist.

Die mindestens eine weitere Komponente bildet sich beispielsweise durch thermische Spaltung von Copolymeren von Alkyl(meth)acrylat und/oder durch thermische Spaltung der in der mindestens einen Polymerzusammensetzung gegebenenfalls enthaltenen zumindest einen weiteren Komponente. Darüber hinaus können sich bei der thermischen Spaltung auch Oligomere von Alkyl(meth)acrylat bilden, diese fallen im Rahmen der vorliegenden Erfindung ebenfalls unter den Begriff "mindestens eine weitere Komponente".

Beispielsweise ist die mindestens eine weitere Komponente ausgewählt aus der Gruppe bestehend aus Styrol, (Meth)acrylsäure, schwefelhaltigen Verbindungen, Oligomeren, Dimeren, C₁-C₄-Alkylsäuren, Di(meth)acrylatdiestern, C₁-C₄-Alkoholen, hochsiedenden Aromaten, Aldehyden und Ketonen, bevorzugt ist die mindestens eine weitere Komponente ausgewählt aus der Gruppe bestehend aus Styrol, (Meth)acrylsäure, schwefelhaltigen Verbindungen, Oligomeren und Dimeren.

Bevorzugt ist daher auch ein Verfahren, bei dem der erste Gasstrom mindestens eine weitere Komponente, ausgewählt aus der Gruppe bestehend aus Styrol, (Meth)acrylsäure, schwefelhaltigen Verbindungen, Oligomeren und Dimeren, enthält.

Unter hochsiedenden Aromaten werden im Rahmen der vorliegenden Erfindung beispielsweise Phenole, Aniline, Benzophenone, Naphthaline und/oder Pyridine verstanden, die jeweils substituiert oder unsubstituiert sein können.

Für Styrol als mindestens eine weitere Komponente gelten die weiter oben beschriebenen Ausführungen und Bevorzugungen für Styrol entsprechend.

Schwefelhaltige Verbindungen leiten sich insbesondere von gegebenenfalls in der mindestens einen Polymerzusammensetzung enthaltenen schwefelhaltigen Reglern ab und sind insbesondere Mercaptane wie Dodecylmercaptan und mehrwertige Mercaptoverbindungen wie Pentaerythroltetrathioglycolat.

Unter Oligomeren werden im Rahmen der vorliegenden Erfindung insbesondere Oligomere des mindestens einen Alkyl(meth)acrylats, von (Meth)acrylsäure und von Mischungen aus diesen verstanden. Der Begriff "Oligomer" umfasst nicht nur höhere Oligomere, sondern auch niedere Oligomere, wie beispielsweise Trimere, Tetramere und Pentamere. Insbesondere umfasst der Begriff "Oligomer" ein Molekül, das aus drei bis zehn Einheiten aufgebaut ist, wobei die Einheiten aus dem mindestens einen Alkyl(meth)acrylat, (Meth)acrylsäure oder Mischungen daraus erhältlich sind.

Unter Dimeren werden im Rahmen der vorliegenden Erfindung insbesondere Dimere des mindestens einen Alkyl(meth)acrylats, von (Meth)acrylsäure und von Mischungen aus diesen verstanden.

Unter C₁-C₄-Alkylsäuren werden aliphatische Carbonsäuren mit 1 bis 4 Kohlenstoffatomen verstanden. Die C₁-C₄-Alkylsäuren können Verzweigungen aufweisen. Beispielsweise ist die C₁-C₄-Alkylsäure ausgewählt aus der Gruppe bestehend aus Ameisensäure, Essigsäure, Propionsäure, Buttersäure und Isobuttersäure.

Unter Di(meth)acrylatdiestern werden nicht nur Ester von (Meth)acrylsäure mit einem Diol verstanden, sondern auch Ester von (Meth)acrylsäure mit einem Polyol, wie beispielsweise einem Triol. Beispielsweise ist der Di(meth)acrylatdiester ausgewählt aus der Gruppe bestehend aus Ethylenglycoldi(meth)acrylat, Propylenglycoldi(meth)acrylat, Trimethylolpropantri(meth)acrylat 1,4-Butandioldi(meth)acrylat und 1,3-Butandioldi(meth)acrylat.

Unter C₁-C₄-Alkoholen werden Alkohole verstanden, die 1 bis 4 Kohlenstoffatome im Alkylrest aufweisen. Der Alkylrest kann linear oder verzweigt sein. Ebenso ist es möglich, dass er mit Heteroatomen substituiert ist. Beispiele für C₁-C₄-Alkohole sind Methanol, Ethanol, Ethylenglycol, n-Butanol und Isobutanol.

Aldehyde sind dem Fachmann als solche bekannt. Beispielsweise sind die Aldehyde ausgewählt aus der Gruppe bestehend aus Formaldehyd, Acetaldehyd, Methacrolein und Acrolein.

Unter Ketonen werden im Rahmen der vorliegenden Erfindung sowohl Monoketone wie beispielsweise Aceton als auch Diketone wie beispielsweise Diacetyl verstanden. Erfindungsgemäße Ketone sind beispielsweise ausgewählt aus der Gruppe bestehend aus Diacetyl, Aceton, Acetylaceton und Methylethylketon.

Beispielsweise enthält der bei der thermischen Spaltung erhaltene erste Gasstrom 60 bis 99 Gew.-%, bevorzugt 80 bis 98 Gew.-%, des mindestens einen Alkyl(meth)acrylats, jeweils bezogen auf das Gesamtgewicht des ersten Gasstroms.

Beispielsweise enthält der bei der thermischen Spaltung erhaltene erste Gasstrom 0,1 bis 10 Gew.-%, bevorzugt 0,5 bis 5 Gew.-%, des mindestens einen weiteren Alkylesters, jeweils bezogen auf das Gesamtgewicht des ersten Gasstroms.

Beispielsweise enthält der bei der thermischen Spaltung erhaltene erste Gasstrom 0,1 bis 30 Gew.-%, bevorzugt 0,5 bis 18 Gew-%, der mindestens einen weiteren Komponente, jeweils bezogen auf das Gesamtgewicht des ersten Gasstroms.

In Schritt b) wird der in Schritt a) erhaltene erste Gasstrom unter Erhalt eines flüssigen ersten Stroms kondensiert. Der flüssige erste Strom enthält das mindestens eine Alkyl(meth)acrylat und den mindestens einen weiteren Alkylester.

Der erste Gasstrom kann nach dem Fachmann bekannten Methoden kondensiert werden, beispielsweise kann die Kondensation an einem Kondensator erfolgen.

Beispielsweise wird der erste Gasstrom in Schritt b) bei einer Temperatur im Bereich von -10 °C bis 100 °C; bevorzugt im Bereich von 0 °C bis 90 °C, besonders bevorzugt im Bereich von 20 °C bis 80 °C, kondensiert.

Der Druck bei der Kondensation des ersten Gasstroms in Schritt b) liegt beispielsweise im Bereich von 0,1 bar bis 1,1 bar, bevorzugt im Bereich von 0,3 bar bis 1 bar und insbesondere bevorzugt im Bereich von 0,4 bar bis 0,8 bar.

Bevorzugt erfolgt die Kondensation des ersten Gasstroms durch einsaugen in zuvor bereits kondensierten und gekühlten ersten Gasstrom, wo der eingesaugte erste Gasstrom dann kondensiert. Ein Teilstrom des kondensierten ersten Gasstroms kann dann als flüssiger erster Strom abgetrennt werden, und erster Gasstrom erneut in den kondensierten und gekühlten ersten Gasstrom eingesaugt werden.

Bei der Kondensation geht der erste Gasstrom von der Gasphase in die flüssige Phase über unter Erhalt des flüssigen ersten Stroms.

Üblicherweise ist das im flüssigen ersten Strom enthaltene mindestens eine Alkyl(meth)acrylat dasselbe mindestens eine Alkyl(meth)acrylat, das im ersten Gasstrom enthalten war. Für das im flüssigen ersten Strom enthaltene mindestens eine Alkyl(meth)acrylat gelten daher die zuvor für das im ersten Gasstrom enthaltene mindestens eine Alkyl(meth)acrylat beschriebenen Ausführungen und Bevorzugungen entsprechend.

Üblicherweise ist der im flüssigen ersten Strom enthaltene mindestens eine weitere Alkylester derselbe mindestens eine weitere Alkylester, der im ersten Gasstrom enthalten war. Daher gelten für den im ersten flüssigen Strom enthaltenen mindestens einen weiteren Alkylester die zuvor beschriebenen Ausführungen und Bevorzugungen wie für den im ersten Gasstrom enthaltenen mindestens einen weiteren Alkylester entsprechend.

Der flüssige erste Strom kann zusätzlich die gegebenenfalls im ersten Gasstrom enthaltene mindestens eine weitere Komponente enthalten. Für die mindestens eine weitere Komponente gelten die zuvor beschriebenen Ausführungen und Bevorzugungen entsprechend.

Erfindungsgemäß bevorzugt wird nach Schritt a) und vor Schritt b) der erste Gasstrom destilliert. Dabei wird ein erster Kopfstrom, der das mindestens eine Alkyl(meth)acrylat und den mindestens einen weiteren Alkylester enthält, und ein erster Sumpfstrom, der mindestens eine von dem mindestens einen Alkyl(meth)acrylat und dem mindestens einen weiteren Alkylester verschiedene Komponente enthält, erhalten. In dieser Ausführungsform wird dann der bei der Destillation erhaltene erste Kopfstrom in Schritt b) kondensiert.

Bevorzugt ist daher auch ein Verfahren, bei dem der erste Gasstrom nach Schritt a) und vor Schritt b) destilliert wird unter Erhalt eines ersten Kopfstroms, der das mindestens eine Alkyl(meth)acrylat und den mindestens einen weiteren Alkylester enthält, und eines ersten Sumpfstroms, der mindestens eine von dem mindestens einen Alkyl(meth)acrylat und dem mindestens einen weiteren Alkylester verschiedene Komponente enthält, wobei der erste Kopfstrom in Schritt b) kondensiert wird.

Der erste Gasstrom kann kondensiert werden bevor er nach Schritt a) und vor Schritt b) destilliert wird. Verfahren zur Kondensation des ersten Gasstroms sind als solche bekannt und beispielsweise weiter oben beschrieben. Unter "Destillation des ersten Gasstroms" wird also im Rahmen der vorliegenden Erfindung nicht nur die Destillation des ersten Gasstroms in der Gasphase bezeichnet, sondern auch und insbesondere die Destillation des ersten Gasstroms nachdem er kondensiert wurde.

Die Destillation des ersten Gasstroms kann nach dem Fachmann bekannten Methoden und in dem Fachmann bekannten Reaktoren erfolgen. Beispielsweise kann der erste Gasstrom in eine Destillationskolonne und/oder in eine Rektifikationskolonne überführt und dort destilliert werden. Bei der Destillation des ersten Gasstroms werden Komponenten, die im ersten Gasstrom enthalten sind und die einen höheren Siedepunkt als das mindestens eine Alkyl(meth)acrylat aufweisen, im ersten Sumpfstrom erhalten, während Komponenten, die im ersten Gasstrom enthalten sind und denselben oder einen niedrigeren Siedepunkt als das mindestens eine Alkyl(meth)acrylat aufweisen, im ersten Kopfstrom erhalten werden.

Die Destillation kann beispielsweise bei einer Temperatur im Bereich von 40 bis 140 °C stattfinden. Bevorzugt liegt die Sumpftemperatur bei der Destillation im Bereich von 95 bis 130 °C, besonders bevorzugt im Bereich von 97 bis 126 °C.

Die Destillation kann beispielsweise bei einem Druck im Bereich von 10 bis 250 mbar stattfinden, bevorzugt im Bereich von 15 bis 150 mbar.

Bei der Destillation des ersten Gasstroms wird ein erster Kopfstrom erhalten. Der erste Kopfstrom enthält das mindestens eine Alkyl(meth)acrylat und den mindestens einen weiteren Alkylester, die bereits im ersten Gasstrom enthalten waren.

Der erste Kopfstrom kann darüber hinaus weitere Komponenten enthalten. Insbesondere enthält der erste Kopfstrom die im ersten Gasstrom enthaltene mindestens eine weitere Komponente, die einen niedrigeren oder genauso hohen Siedepunkt aufweist wie das mindestens eine Alkyl(meth)acrylat und der mindestens eine weitere Alkylester. Daher enthält der erste Kopfstrom üblicherweise mindestens eine weitere Komponente ausgewählt aus der Gruppe bestehend aus Methylpropionat, Ethylpropionat, Methylisobutyrat, Methylpivalat, Methyl-3-methoxyisobutyrat und Dicarbonsäurediestern.

Beispielsweise enthält der bei der Destillation erhaltene erste Kopfstrom 60 bis 99 Gew.-%, bevorzugt 80 bis 98 Gew.-%, des mindestens einen Alkyl(meth)acrylats, jeweils bezogen auf das Gesamtgewicht des ersten Kopfstroms.

Beispielsweise enthält der bei der Destillation erhaltene erste Kopfstrom 0,1 bis 10 Gew.-%, bevorzugt 0,5 bis 5 Gew.-%, des mindestens einen weiteren Alkylesters, jeweils bezogen auf das Gesamtgewicht des ersten Kopfstroms.

Beispielsweise enthält der bei der Destillation erhaltene erste Kopfstrom 0,1 bis 30 Gew.-%, bevorzugt 0,5 bis 18 Gew-%, der mindestens einen weiteren Komponente, jeweils bezogen auf das Gesamtgewicht des ersten Kopfstroms.

Außerdem wird ein erster Sumpfstrom erhalten. Unter einem "Sumpfstrom" wird im Rahmen der vorliegenden Erfindung nicht nur ein Sumpfprodukt verstanden, das kontinuierlich aus der Destillation, insbesondere aus dem Reaktor, entfernt wird, sondern auch ein Sumpfprodukt, das, beispielsweise bei diskontinuierlicher Fahrweise, als Destillationsrückstand im Reaktor verbleibt und erst zu einem späteren Zeitpunkt aus dem Reaktor entfernt wird.

Der erste Sumpfstrom enthält mindestens eine von dem mindestens einen Alkyl(meth)acrylat und dem mindestens einen weiteren Alkylester verschiedene Komponente. Diese Komponente ist üblicherweise mindestens eine der im ersten Gasstrom enthaltenen mindestens einen weiteren Komponenten. Insbesondere weist diese mindestens eine weitere Komponente üblicherweise eine höhere Siedetemperatur auf als das im ersten Gasstrom enthaltene mindestens eine Alkyl(meth)acrylat und als der im ersten Gasstrom enthaltene mindestens eine weitere Alkylester. Beispielsweise ist diese mindestens eine weitere Komponente ausgewählt aus der Gruppe bestehend aus schwefelhaltigen Verbindungen, hochsiedende Aromaten, Oligomeren und Dimeren. Für die schwefelhaltigen Verbindungen, die hochsiedenden Aromaten, die Oligomere und die Dimere gelten die zuvor für die in dem ersten Gasstrom enthaltenen schwefelhaltigen Verbindungen, die hochsiedenden Aromaten, die Oligomere und die Dimere beschriebenen Ausführungen und Bevorzugungen entsprechend.

Der erste Sumpfstrom enthält daher bevorzugt schwefelhaltige Verbindungen, hochsiedende Aromaten, Oligomere und/oder Dimere.

In Schritt c) wird der in Schritt b) erhaltene flüssige erste Strom mit einem weiteren Strom gemischt unter Erhalt eines Mischstroms. Der weitere Strom enthält mindestens ein weiteres Alkyl(meth)acrylat und ist Teil eines C₂-C₁₈-Alkyl(meth)acrylat-Herstellverfahrens. Der erhaltene Mischstrom umfasst den flüssigen ersten Strom und den weiteren Strom.

Beispielsweise werden bis zu 90 Gew.-%, bevorzugt bis zu 60 Gew.-% des flüssigen ersten Stroms mit dem weiteren Strom gemischt bezogen auf das Gesamtgewicht des erhaltenen Mischstroms.

Der weitere Strom, mit dem der flüssige erste Strom gemischt wird, enthält mindestens ein weiteres Alkyl(meth)acrylat. "Mindestens ein weiteres Alkyl(meth)acrylat" bedeutet im Rahmen der vorliegenden Erfindung sowohl genau ein weiteres Alkyl(meth)acrylat als auch eine Mischung aus zwei oder mehreren weiteren Alkyl(meth)acrylaten. Genau ein weiteres Alkyl(meth)acrylat ist bevorzugt.

Für das im weiteren Strom enthaltene mindestens eine weitere Alkyl(meth)acrylat gelten die zuvor für das im ersten Gasstrom enthaltene mindestens eine Alkyl(meth)acrylat beschriebenen Ausführungen und Bevorzugungen entsprechend. Das mindestens eine weitere Alkyl(meth)acrylat ist daher beispielsweise ausgewählt aus der Gruppe bestehend aus C₁-C₄-Alkyl(meth)acrylaten. Insbesondere bevorzugt ist das im weiteren Strom enthaltene Alkyl(meth)acrylat Methyl(meth)acrylat.

Das im flüssigen ersten Strom enthaltene mindestens eine Alkyl(meth)acrylat umfasst bevorzugt dasselbe Alkyl(meth)acrylat wie das Alkyl(meth)acrylat, das das mindestens eine weitere Alkyl(meth)acrylat des weiteren Stroms umfasst.

Es ist erfindungsgemäß bevorzugt, dass, bezogen auf das Gesamtgewicht des im flüssigen ersten Stroms enthaltenen mindestens einen Alkyl(meth)acrylats, mindestens 80 Gew.-%, bevorzugt mindestens 90 Gew.-% des im flüssigen ersten Stroms enthaltenen mindestens einen Alkyl(meth)acrylats, dasselbe Alkyl(meth)acrylat ist wie das im weiteren Strom enthaltene mindestens eine weitere Alkyl(meth)acrylat.

Der weitere Strom ist Teil eines C₂-C₁₈-Alkyl(meth)acrylat-Herstellverfahrens. Unter einem "C₂-C₁₈-Alkyl(meth)acrylat-Herstellverfahren" wird im Rahmen der vorliegenden Erfindung ein Verfahren zur Herstellung von C₂-C₁₈-Alkyl(meth)acrylaten durch Umesterung von Alkyl(meth)acrylaten verstanden. Für die Alkyl(meth)acrylate gelten die zuvor beschriebenen Ausführungen und Bevorzugungen entsprechend. Dementsprechend ist ein C₂-C₁₈-Alkyl(meth)acrylat-Herstellverfahren bevorzugt ein Verfahren zur Herstellung von C₂-C₁₈-Alkyl(meth)acrylaten durch Umesterung von C₁-C₄-Alkyl(meth)acrylaten. Es versteht sich von selbst, dass die Alkyl(meth)acrylate, die in dem Verfahren umgeestert werden verschieden sind von den C₂-C₁₈-Alkyl(meth)acrylaten, die hergestellt werden. Die Alkyl(meth)acrylate, die umgeestert werden, sind üblicherweise das mindestens eine weitere Alkyl(meth)acrylat, das im weiteren Strom enthalten ist, und das mindestens eine Alkyl(meth)acrylat, das im flüssigen ersten Strom enthalten ist.

Der flüssige erste Strom kann nach dem Fachmann bekannten Methoden mit dem weiteren Strom gemischt werden. Beispielsweise und erfindungsgemäß bevorzugt wird der flüssige erste Strom in eine Anlage zur Herstellung eines C₂-C₁₈-Alkyl(meth)acrylats eingespeist, die den weiteren Strom enthält. Der erhaltene Mischstrom ist dann vorzugsweise ebenfalls in der Anlage zur Herstellung eines C₂-C₁₈-Alkyl(meth)acrylats enthalten. Der Mischstrom ist dann vorzugsweise ebenfalls Teil des C₂-C₁₈-Alkyl(meth)acrylat-Herstellverfahrens.

Bevorzugt ist daher auch ein Verfahren, bei dem der in Schritt c) erhaltene Mischstrom Teil des C₂-C₁₈-Alkyl(meth)acrylat-Herstellverfahrens ist.

Der Mischstrom umfasst den flüssigen ersten Strom und den weiteren Strom. Der Mischstrom umfasst daher üblicherweise dieselben Komponenten, die im flüssigen ersten Strom und in dem weiteren Strom enthalten waren. Daher umfasst der Mischstrom üblicherweise das mindestens eine Alkyl(meth)acrylat, den mindestens einen weiteren Alkylester, das mindestens eine weitere Alkyl(meth)acrylat sowie gegebenenfalls die mindestens eine weitere Komponente.

Der flüssige erste Strom kann an beliebiger Stelle des C₂-C₁₈-Alkyl(meth)acrylat-Herstellverfahrens mit dem weiteren Strom gemischt werden. Beispielsweise wird der flüssige erste Strom mit dem weiteren Strom vor dem weiter unten beschriebenen Reaktor in der Anlage zur Herstellung eines C₂-C₁₈-Alkyl(meth)acrylats gemischt. Ebenso ist es möglich, dass der flüssige erste Strom mit dem weiteren Strom im weiter unten beschriebenen Reaktor gemischt wird.

Wird der flüssige erste Strom mit dem weiteren Strom in dem weiter unten beschriebenen Reaktor gemischt, so werden die Schritte c) und d) üblicherweise zeitgleich durchgeführt.

Bevorzugt ist daher auch ein Verfahren, bei dem die Schritte c) und d) zeitgleich durchgeführt werden.

In Schritt d) wird der in Schritt c) erhaltene Mischstrom mit einem C₂-C₁₈-Alkohol in Gegenwart eines Katalysators umgesetzt. Dabei wird ein Produktstrom erhalten, der das mindestens eine Alkyl(meth)acrylat, das mindestens eine weitere Alkyl(meth)acrylat, das C₂-C₁₈-Alkyl(meth)acrylat, den Katalysator, den mindestens einen Alkylester, den C₂-C₁₈-Alkohol und mindestens einen weiteren Alkohol enthält.

Im Rahmen der vorliegenden Erfindung umfasst der Begriff "ein C₂-C₁₈-Alkohol" sowohl genau einen C₂-C₁₈-Alkohol als auch eine Mischung aus zwei oder mehreren C₂-C₁₈-Alkoholen. Erfindungsgemäß bevorzugt ist genau ein C₂-C₁₈-Alkohol.

Unter einem "C₂-C₁₈-Alkohol" wird im Rahmen der vorliegenden Erfindung ein Alkohol verstanden, der 2 bis 18 Kohlenstoffatome im Alkylrest aufweist. Der Alkylrest kann zyklisch oder linear sein, ebenso sind Verzweigungen möglich. Der Alkylrest mit 2 bis 18 Kohlenstoffatomen kann auch mit Heteroatomen innerhalb des Alkylrests substituiert sein. Unter C₂-C₁₈-Alkoholen werden im Rahmen der vorliegenden Erfindung auch Alkohole verstanden, die einen aromatischen Rest im Alkylrest aufweisen, wobei ein solcher C₂-C₁₈-Alkohol dann im Alkylrest und dem aromatischen Rest insgesamt 2 bis 18 Kohlenstoffatome aufweist. Ein Beispiel für einen solchen C₂-C₁₈-Alkohol mit aromatischem Rest im Alkylrest ist Benzylalkohol. Der C₂-C₁₈-Alkohol kann genau eine Hydroxylgruppe (OH-Gruppe) aufweisen. Ebenso kann er zwei oder mehrere Hydroxylgruppen aufweisen. Weist der C₂-C₁₈-Alkohol zwei oder mehrere Hydroxylgruppen auf, so wird der Alkohol auch als Polyol bezeichnet. Es ist daher möglich, dass der C₂-C₁₈-Alkohol ein Polyol umfasst.

Erfindungsgemäß bevorzugt ist daher auch ein Verfahren, bei dem der C₂-C₁₈-Alkohol in Schritt d) ein Polyol umfasst.

Enthält der C₂-C₁₈-Alkohol genau zwei Hydroxylgruppen, so wird er auch als Diol bezeichnet. Enthält der C₂-C₁₈-Alkohol genau drei Hydroxylgruppen, so wird er auch als Triol bezeichnet.

Der C₂-C₁₈-Alkohol ist bevorzugt ein C₄-C₁₂-Alkohol.

Beispiele für C₂-C₁₈-Alkohole sind Ethanol, Propanol, Isopropanol, n-Butanol, tert.-Butanol, Isobutanol, Pentanol, Cyclohexanol, Hexanol, Heptanol, Octanol, Isooctanol, Isodecanol, 2-Ethylhexanol, Isoborneol, Benzylalkohol, Tetrahydrofurfurol, 3,3,4-Trimethyl-Cyclohexanol, Phenylethanol, tert.-Butylaminoethanol, Diethylaminoethanol, Ethylenglycol, Ethylentriglycol, Methylentriglycol, Butyldiglycol, 1,3-Butandiol, 1,4-Butandiol, Trimethylolpropan und Isopropylidenglycerin.

Bevorzugt ist der C₂-C₁₈-Alkohol in Schritt d) ausgewählt aus der Gruppe bestehend aus Ethanol, n-Butanol, Isobutanol, 2-Ethylhexanol, tert.-Butanol, Isodecanol und Cyclohexanol.

Insbesondere bevorzugt ist der C₂-C₁₈-Alkohol in Schritt d) ausgewählt aus der Gruppe bestehend aus n-Butanol, Isobutanol und 2-Ethylhexanol.

Beispielsweise liegt das Stoffmengenverhältnis (molare Verhältnis) des Mischstroms, insbesondere des im Mischstrom enthaltenen mindestens einen Alkyl(meth)acrylats und mindestens einen weiteren Alkyl(meth)acrylats, zu dem C₂-C₁₈-Alkohol im Reaktor im Bereich von 2 zu 1 bis 1 zu 2, bevorzugt im Bereich 1,1 zu 1 bis 1,2 zu 1.

Es versteht sich von selbst, dass sich das Stoffmengenverhältnis des Mischstroms, insbesondere des im Mischstrom enthaltenen mindestens einen Alkyl(meth)acrylats und mindestens einen weiteren Alkyl(meth)acrylats, zu dem C₂-C₁₈-Alkohol auf das Stoffmengenverhältnis bezieht, bevor der Mischstrom mit dem C₂-C₁₈-Alkohol umgesetzt wurde. Dem Fachmann ist klar, dass sich das Stoffmengenverhältnis während der Umsetzung ändern kann, beispielsweise durch Nebenreaktionen und/oder durch Destillation einer Komponente.

Der Mischstrom wird in Schritt d) in Gegenwart eines Katalysators umgesetzt. "Ein Katalysator" bedeutet im Rahmen der vorliegenden Erfindung sowohl genau ein Katalysator als auch eine Mischung aus zwei oder mehreren Katalysatoren. Genau ein Katalysator ist erfindungsgemäß bevorzugt.

Dem Fachmann ist klar, dass sich während der Umsetzung in Schritt d) die Zusammensetzung des Katalysators ändern kann, beispielsweise durch Umesterung. Daher ist es möglich, dass die Umsetzung in Schritt d) in Gegenwart von genau einem Katalysator beginnt, dass aber im Laufe der Umsetzung sich die Zusammensetzung des ersten Katalysators ändern kann, sodass während der Umsetzung in Schritt d) eine Mischung aus zwei oder mehreren ersten Katalysatoren gebildet werden kann und die Umsetzung in Schritt d) dann in Gegenwart einer Mischung von zwei oder mehreren Katalysatoren stattfindet.

Beispielsweise erfolgt die Umsetzung in Schritt d) in Gegenwart von 0,01 bis 5 Gew.-% des Katalysators, bevorzugt im Bereich von 0,1 bis 0,5 Gew.-% des Katalysators, jeweils bezogen auf das Gesamtgewicht an mindestens einem Alkyl(meth)acrylat und mindestes einem weiteren Alkyl(meth)acrylat.

Als Katalysator eignen sich alle dem Fachmann bekannten Katalysatoren, die die Umsetzung der im Mischstrom enthaltenen Komponenten, insbesondere des im Mischstrom enthaltenen mindestens einen Alkyl(meth)acrylats und mindestens einem weiteren Alkyl(meth)acrylats, mit dem C₂-C₁₈-Alkohol katalysieren.

Bevorzugt ist der Katalysator ausgewählt aus der Gruppe bestehend aus Metallsalzen und organischen Verbindungen, jeweils von Metallen ausgewählt aus der Gruppe bestehend aus Zinn, Titan, Zirkon, Alkalimetallen und Erdalkalimetallen.

Bevorzugt ist daher auch ein Verfahren, bei dem der Katalysator ausgewählt ist aus der Gruppe bestehend aus Metallsalzen und organischen Verbindungen, jeweils von Metallen ausgewählt aus der Gruppe bestehend aus Zinn, Titan, Zirkon, Alkalimetallen und Erdalkalimetallen.

Geeignete Metallsalze von Metallen ausgewählt aus der Gruppe bestehend aus Zinn, Titan, Zirkon, Alkalimetallen und Erdalkalimetallen sind dem Fachmann bekannt und beispielsweise Lithiumhydroxid, Calciumhydroxid, Calciumoxid, Lithiumamid, Lithiumchlorid, Natriumhydroxid und/oder Kaliumhydroxid, bevorzugt sind Alkalimetallchloride und/oder Alkalimetallhydroxide, besonders bevorzugt sind Mischungen aus Alkalimetallchloriden und/oder Alkalimetallhydroxiden mit Erdalkalimetalloxiden.

Geeignete organische Verbindungen von Metallen ausgewählt aus der Gruppe bestehend aus Zinn, Titan, Zirkon, Alkalimetallen und Erdalkalimetallen sind dem Fachmann bekannt und beispielsweise Dibutylzinnoxid, Dioctylzinnoxid, Tetraisopropyltitanat und/oder Zirkonacetonylacetonat., bevorzugt ist Tetraisopropyltitanat und/oder Zirkonacetonylacetonat.

Die Umsetzung in Schritt d) findet beispielsweise bei einer Temperatur im Bereich von 80 °C bis 160 °C statt, bevorzugt bei einer Temperatur im Bereich von 110 °C bis 135 °C.

Die Umsetzung in Schritt d) kann in dem Fachmann bekannten Reaktoren für Umesterungen stattfinden. Umesterungen und Reaktoren hierfür sind beispielsweise in EP 1 583 733 und DE 1 200 171 beschrieben.

Beispielsweise kann die Umsetzung in Schritt d) in einem Reaktor stattfinden, der einen Brüdenabzug zu einer ersten Destillationskolonne umfasst. Diese erste Destillationskolonne wird auch als Azeotrop-Destillationskolonne bezeichnet.

Die Umsetzung in Schritt d) kann in beliebigen Reaktoren erfolgen. Sie kann kontinuierlich erfolgen, ebenso ist eine diskontinuierliche Umsetzung möglich. Bevorzugt erfolgt die Umsetzung in Schritt d) kontinuierlich. Unter "kontinuierlich" wird in diesem Zusammenhang verstanden, dass der Mischstrom oder der flüssige erste Strom und der weitere Strom dem Reaktor kontinuierlich zugeführt werden, während gleichzeitig der Produktstrom kontinuierlich aus dem Reaktor abgeführt wird.

Die Umsetzung in Schritt d) kann in Gegenwart weiterer Komponenten erfolgen. Derartige weitere Komponenten sind als solche bekannt und beispielsweise Stabilisatoren.

Als Stabilisatoren eignen sich insbesondere Verbindungen, die die Polymerisation des Mischstroms, insbesondere des darin enthaltenen mindestens einen Alkyl(meth)acrylats und des mindestens einen weiteren Alkyl(meth)acrylats, inhibieren. Die Stabilisatoren werden daher auch als Polymerisationsinhibitoren bezeichnet.

Geeignete Stabilisatoren sind beispielsweise Hydrochinonmonomethylether in Verbindung mit Sauerstoff, Phenol, Hydrochinon, Nitrophenol und/oder Butylhydroxytoluol.

Zu den bevorzugt eingesetzten Stabilisatoren zählen insbesondere Phenolverbindungen, wie zum Beispiel Hydrochinone, Hydrochinonether, wie Hydrochinonmonomethylether, tert-Butylhydrochinon, 2,6-Di-tert-butylhydrochinon, 2,5-Di-tert-butylhydrochinon, 2,4-Dimethyl-6-tert-butylphenol oder Di-tert-butylbrenzcatechin; p-Phenylendiamine, wie zum Beispiel N,N'-Diphenyl-p-phenylendiamin, N,N'-Di-2-naphthyl-p-phenylendiamin, N,N'-Di-p-tolyl-p-phenylendiamin, N-1,3-Dimethylbutyl-N'-phenyl-p-phenylendiamin und N-1,4-Dimethylpentyl-N'-phenyl-p-phenylendiamin; Amine, wie zum Beispiel Thiodiphenylamin und Phenothiazin; Kupferdialkyldithiocarbamate, wie zum Beispiel Kupferdimethyldithiocarbamate, Kupferdiethyldithiocarbamate und Kupferdibutyldithiocarbamate; Nitrosoverbindungen, wie zum Beispiel Nitrosodiphenylamin, Isoamylnitrit, N-Nitrosocyclohexylhydroxylamin, N-Nitroso-N-phenyl-N-hydroxylamin und deren Salze; und N-Oxylverbindungen, wie zum Beispiel 2,2,4,4-Tetramethylazetidin-1-oxyl, 2,2-Dimethyl-4,4-dipropylazetidin-1-oxyl, 2,2,5,5-Tetramethylpyrrolidin-1-oxyl, 2,2,5,5-Tetramethyl-3-oxopyrrolidin-1-oxyl, 2,2,6,6-Tetramethylpiperidin-1-oxyl, 4-Hydroxy-2,2,6,6-tetramethylpiperidin-1-oxyl, 6-Aza-7,7-dimethylspiro[4,5]decan-6-oxyl, 2,2,6,6-Tetramethyl-4-acetoxypiperidin-1-oxyl und 2,2,6,6-Tetramethyl-4-benzoyloxypiperidin-1-oxyl; Methylenblau, Nigrosin Base BA, 1,4-Benzochinon, sterisch gehinderte Phenole, beispielsweise 2,4-dimethyl-6-tert-butylphenol und/oder Tocopherol-Verbindungen, vorzugsweise α-Tocopherol.

Die Stabilisatoren können einzeln oder in Form von Mischungen eingesetzt werden und sind im Allgemeinen kommerziell erhältlich. Für weitere Details wird auf die gängige Fachliteratur, insbesondere auf das Römpp-Lexikon Chemie; Herausgeber: J. Falbe, M. Regitz; Stuttgart, New York; 10. Auflage (1996); Stichwort "Antioxidantien" und die an dieser Stelle zitierten Literaturstellen verwiesen.

Beispielsweise erfolgt die Umsetzung in Schritt d) in Gegenwart von 1 bis 5000 Gew.-ppm Stabilisator, bevorzugt 5 bis 1000 Gew.-ppm, besonders bevorzugt von 10 bis 100 Gew.-ppm, jeweils bezogen auf das Gesamtgewicht an Mischstrom, insbesondere an dem mindestens einen Alkyl(meth)acrylat und dem mindestens einen weiteren Alkyl(meth)acrylat.

In Schritt d) wird ein Produktstrom erhalten. Der Produktstrom enthält das mindestens eine Alkyl(meth)acrylat, das mindestens eine weitere Alkyl(meth)acrylat, das C₂-C₁₈-Alkyl(meth)acrylat, den Katalysator, den mindestens einen Alkylester, den C₂-C₁₈-Alkohol und mindestens einen weiteren Alkohol.

Es versteht sich von selbst, dass das mindestens eine Alkyl(meth)acrylat, das mindestens eine weitere Alkyl(meth)acrylat und der mindestens eine Alkylester, die im Produktstrom enthalten sind, dasselbe mindestens eine Alkyl(meth)acrylat, mindestens eine weitere Alkyl(meth)acrylat und mindestens eine Alkylester sind, die im Mischstrom enthalten waren und (noch) nicht umgesetzt wurden. Es versteht sich weiterhin von selbst, dass der im Produktstrom enthaltene C₂-C₁₈-Alkohol derselbe C₂-C₁₈-Alkohol ist, mit dem der Mischstrom umgesetzt worden ist. Der im Produktstrom enthaltene Katalysator ist derselbe Katalysator, in dessen Gegenwart der Mischstrom mit dem C₂-C₁₈-Alkohol umgesetzt worden ist

Bei der Umsetzung des Mischstroms, insbesondere des mindestens einen Alkyl(meth)acrylats und des mindestens einen weiteren Alkyl(meth)acrylats, mit dem C₂-C₁₈-Alkohol in Gegenwart des Katalysators bildet sich das C₂-C₁₈-Alkyl(meth)acrylat. Gleichzeitig bildet sich mindestens ein weiterer Alkohol des Alkylrests des mindestens einen Alkyl(meth)acrylats und des mindestens einen weiteren Alkyl(meth)acrylats. Der gebildete mindestens eine weitere Alkohol ist daher üblicherweise ein C₁-C₁₈-Alkohol, bevorzugt ein C₁-C₁₂-Alkohol, insbesondere bevorzugt ein C₁-C₄-Alkohol.

Es versteht sich von selbst, dass der mindestens eine weitere Alkohol von dem C₂-C₁₈-Alkohol verschieden ist.

Ebenso ist das C₂-C₁₈-Alkyl(meth)acrylat von dem mindestens einen Alkyl(meth)acrylat und dem mindestens einen weiteren Alkyl(meth)acrylat verschieden.

Beispielsweise enthält der in Schritt d) erhaltene Produktstrom
1 bis 30 Gew.-% mindestens ein Alkyl(meth)acrylat und mindestens ein weiteres Alkyl(meth)acrylat,
47 bis 88 Gew.-% C₂-C₁₈-Alkyl(meth)acrylat,
0,05 bis 0,4 Gew.-% Katalysator,
0,01 bis 1 Gew.-% mindestens ein Alkylester,
2 bis 22 Gew.-% C₂-C₁₈-Alkohol und
0,1 bis 1,1 Gew.-% mindestens ein weiterer Alkohol,
jeweils bezogen auf das Gesamtgewicht des Produktstroms.

Bevorzugt werden bei der Umsetzung in Schritt d) ein zweiter Kopfstrom und ein zweiter Sumpfstrom erhalten.

Der zweite Kopfstrom enthält dann das mindestens eine Alkyl(meth)acrylat, das mindestens eine weitere Alkyl(meth)acrylat, den mindestens einen Alkylester und den mindestens einen weiteren Alkohol.

Der zweite Sumpfstrom umfasst den Produktstrom.

Bevorzugt ist daher auch ein Verfahren, bei dem bei der Umsetzung in Schritt d) ein zweiter Kopfstrom, der das mindestens eine Alkyl(meth)acrylat, das mindestens eine weitere Alkyl(meth)acrylat, den mindestens einen Alkylester und den mindestens einen weiteren Alkohol enthält, und ein zweiter Sumpfstrom, der den Produktstrom umfasst, erhalten wird.

Üblicherweise bilden sich der zweite Kopfstrom und der zweite Sumpfstrom durch Destillation während der Umsetzung in Schritt d). Wie vorstehend ausgeführt, kann der Reaktor, in dem die Umsetzung in Schritt d) stattfindet, beispielsweise eine erste Destillationskolonne umfassen. Die Destillation während der Umsetzung in Schritt d) findet dann vorzugsweise in der ersten Destillationskolonne statt.

Der zweite Kopfstrom kann zumindest teilweise aus der Umsetzung in Schritt d) entfernt werden. Dies ist insbesondere vorteilhaft, da so das Reaktionsgleichgewicht der Umsetzung in Schritt d) hin zum Produktstrom verschoben wird.

Der erhaltene zweite Kopfstrom kann zumindest teilweise in Schritt c) und/oder d) rückgeführt werden.

Darüber hinaus ist es möglich, dass der zweite Kopfstrom zumindest teilweise einem Verfahren zur Herstellung eines C₁-C₂-Alkyl(meth)acrylats, insbesondere Methyl(meth)acrylat, zugeführt wird.

Bevorzugt ist daher auch ein Verfahren, bei dem der erhaltene zweite Kopfstrom in Schritt c) und/oder Schritt d) rückgeführt wird und/oder einem Verfahren zur Herstellung eines C₁-C₂-Alkyl(meth)acrylats zugeführt wird.

Verfahren zur Herstellung von C₁-C₂-Alkyl(meth)acrylaten, insbesondere von Methyl(meth)acrylat, sind dem Fachmann als solche bekannt. Üblicherweise werden diese mittels diverser Verfahren, die von C₂-, C₃- oder C₄-Bausteinen ausgehen, hergestellt.

In Schritt e) wird das C₂-C₁₈-Alkyl(meth)acrylat aus dem Produktstrom abgetrennt.

Das C₂-C₁₈-Alkyl(meth)acrylat kann nach dem Fachmann bekannten Methoden aus dem Produktstrom abgetrennt werden. Vorzugsweise wird das C₂-C₁₈-Alkyl(meth)acrylat durch Destillation aus dem Produktstrom abgetrennt.

Beispielsweise umfasst Schritt e) den folgenden Schritt e1):
e1) Destillation des Produktstroms unter Erhalt eines dritten Kopfstroms, der das mindestens eine Alkyl(meth)acrylat, das mindestens eine weitere Alkyl(meth)acrylat, den mindestens einen Alkylester, den C₂-C₁₈-Alkohol und den mindestens einen weiteren Alkohol enthält, und eines dritten Sumpfstroms, der das C₂-C₁₈-Alkyl(meth)acrylat, Reste des mindestens einen Alkylesters und den Katalysator enthält.

Bevorzugt ist daher auch ein Verfahren, bei dem Schritt e) den folgenden Schritt e1) umfasst:
e1) Destillation des Produktstroms unter Erhalt eines dritten Kopfstroms, der das mindestens eine Alkyl(meth)acrylat, das mindestens eine weitere Alkyl(meth)acrylat, den mindestens einen Alkylester, den C2-C18-Alkohol und den mindestens einen weiteren Alkohol enthält, und eines dritten Sumpfstroms, der das C2-C18-Alkyl(meth)acrylat, Reste des mindestens einen Alkylesters und den Katalysator enthält.

Die Destillation in Schritt e1) findet beispielsweise in einer zweiten Destillationskolonne statt. Die Komponenten des dritten Kopfstroms weisen eine niedrigere Siedetemperatur als das C₂-C₁₈-Alkyl(meth)acrylat auf und werden von dem C₂-C₁₈-Alkyl(meth)acrylat abgetrennt. Daher wird die zweite Destillationskolonne auch als Niedrigsieder-Destillationskolonne bezeichnet.

Üblicherweise wird die zweite Mischung im Anschluss an Schritt e), vorzugsweise kontinuierlich, in die zweite Destillationskolonne überführt und dort gemäß Schritt e1) destilliert.

Die Destillation in Schritt e1) erfolgt üblicherweise bei einer Temperatur im Bereich von 45 °C bis 130 °C. Bevorzugt liegt die Sumpftemperatur bei der Destillation im Bereich von 50 °C bis 180 °C, besonders bevorzugt im Bereich von 100 °C bis 150 °C.

Die Destillation in Schritt e1) erfolgt vorzugsweise bei vermindertem Druck, insbesondere bei einem Druck im Bereich von 10 mbar bis 200 mbar.

Der in Schritt e1) erhaltene dritte Kopfstrom kann beispielsweise in Schritt c) und/oder d) des erfindungsgemäßen Verfahrens rückgeführt werden. Dadurch können das im dritten Kopfstrom enthaltene mindestens eine Alkyl(meth)acrylat, das mindestens eine weitere Alkyl(meth)acrylat und der C₂-C₁₈-Alkohol gemäß Schritt d) umgesetzt werden, sodass Ressourcen gespart werden können und der Gesamtumsatz des erfindungsgemäßen Verfahrens weitestgehend vollständig verläuft.

Es ist darüber hinaus möglich, den dritten Kopfstrom zumindest teilweise einem Verfahren zur Herstellung eines C₁-C₂-Alkyl(meth)acrylats zuzuführen.

Bevorzugt ist daher auch ein Verfahren, bei dem der dritte Kopfstrom in Schritt c) und/oder Schritt d) rückgeführt wird und/oder einem Verfahren zur Herstellung eines C₁-C₂-Alkyl(meth)acrylats zugeführt wird.

Bevorzugt wird der dritte Kopfstrom in Schritt c) und/oder d) des erfindungsgemäßen Verfahrens rückgeführt.

Der in Schritt e1) erhaltene dritte Sumpfstrom enthält vorzugsweise mehr als 98 Gew.-% C₂-C₁₈-Alkyl(meth)acrylat, bezogen auf das Gesamtgewicht des dritten Sumpfstroms.

Erfolgt die Umsetzung in Schritt d) in Anwesenheit mindestens eines Polymerisationsinhibitors, so enthält der dritte Sumpfstrom üblicherweise ebenfalls den mindestens einen Polymerisationsinhibitor.

Es ist erfindungsgemäß bevorzugt, dass im Anschluss an Schritt e1) der folgende Schritt e2) durchgeführt wird:
e2) Destillation des in Schritt e1) erhaltenen dritten Sumpfstroms unter Erhalt eines vierten Kopfstroms, der das C₂-C₁₈-Alkyl(meth)acrylat enthält, und eines vierten Sumpfstroms, der die Reste des mindestens einen Alkylesters und den Katalysator enthält.

Erfindungsgemäß bevorzugt ist daher auch ein Verfahren, bei dem im Anschluss an Schritt e1) der folgende Schritt e2) durchgeführt wird:
e2) Destillation des in Schritt e1) erhaltenen dritten Sumpfstroms unter Erhalt eines vierten Kopfstroms, der das C2-C18-Alkyl(meth)acrylat enthält, und eines vierten Sumpfstroms, der die Reste des mindestens einen Alkylesters und den Katalysator enthält.

Die Destillation in Schritt e2) findet beispielsweise in einer dritten Destillationskolonne statt. Das C₂-C₁₈-Alkyl(meth)acrylat wird im vierten Kopfstrom erhalten. Die Komponenten des vierten Sumpfstroms weisen eine höhere Siedetemperatur als das C₂-C₁₈-Alkyl(meth)acrylat auf und werden von dem C₂-C₁₈-Alkyl(meth)acrylat abgetrennt. Daher wird die dritte Destillationskolonne auch als Hochsieder-Destillationskolonne bezeichnet.

Üblicherweise wird der in Schritt e1) erhaltene dritte Sumpfstrom kontinuierlich in die dritte Destillationskolonne überführt und dort gemäß Schritt e2) destilliert.

Die Destillation in Schritt e2) erfolgt üblicherweise bei einer Temperatur im Bereich von 85 bis 120 °C.

Die Destillation in Schritt e2) erfolgt vorzugsweise bei vermindertem Druck, insbesondere bei einem Druck im Bereich von 20 mbar bis 200 mbar.

Die Destillation in Schritt e2) kann beispielsweise eine Filmverdampfung umfassen. Geeignete Filmverdampfer sind als solche bekannt und beispielsweise ausgewählt aus der Gruppe bestehend aus Fallfilmverdampfern, Dünnschichtverdampfern und Kurzwegverdampfern.

Der in Schritt e2) erhaltene vierte Sumpfstrom enthält den Katalysator. Bevorzugt wird der vierte Sumpfstrom in Schritt d) rückgeführt. Dies macht das erfindungsgemäße Verfahren besonders wirtschaftlich.

Bevorzugt ist daher auch ein Verfahren, bei dem der vierte Sumpfstrom in Schritt d) rückgeführt wird.

Der vierte Sumpfstrom kann vor seiner Rückführung in Schritt d) aufgereinigt werden, beispielsweise in einer Vakuum-Eindampfstufe. Derartige Vakuum-Eindampfstufen sind als solche bekannt und beispielsweise Filmverdampfer wie Fallfilmverdampfer, Dünnschichtverdampfer und/oder Kurzwegverdampfer.

In der Vakuum-Eindampfstufe können zudem gegebenenfalls im vierten Sumpfstrom enthaltene Reste von C₂-C₁₈-Alkyl(meth)acrylat aus dem vierten Sumpfstrom entfernt werden, was die Ausbeute an C₂-C₁₈-Alkyl(meth)acrylat weiter erhöht.

In Schritt e) wird das C₂-C₁₈-Alkyl(meth)acrylat abgetrennt. Insbesondere bevorzugt wird das C₂-C₁₈-Alkyl(meth)acrylat im vierten Kopfstrom in Schritt e2) erhalten.

Unter dem Begriff "C₂-C₁₈-Alkyl(meth)acrylat" werden Alkylester von (Meth)acrylsäure verstanden, die 2 bis 18 Kohlenstoffatome im Alkylrest aufweisen. Der Alkylrest kann zyklisch oder linear sein, ebenso sind Verzweigungen möglich. Der Alkylrest mit 2 bis 18 Kohlenstoffatomen kann auch mit Heteroatomen substituiert sein und insbesondere Hydroxylgruppen (OH-Gruppen) aufweisen.

Beispielsweise ist das C₂-C₁₈-Alkyl(meth)acrylat ausgewählt aus der Gruppe bestehend aus Ethyl(meth)acrylat, n-Butyl(meth)acrylat, Isobutyl(meth)acrylat, 2-Ethylhexyl(meth)acrylat, Stearyl(meth)acrylat, Cyclohexyl(meth)acrylat, Benzyl(meth)acrylat, Isodecyl(meth)acrylat und Lauryl(meth)acrylat.

Es versteht sich von selbst, dass sich der Alkylrest mit den 2 bis 18 Kohlenstoffatomen von dem in Schritt d) umgesetzten C₂-C₁₈-Alkohol ableitet.

Wird also beispielsweise als C₂-C₁₈-Alkohol in Schritt d) Isobutanol umgesetzt, so ist das erhaltene C₂-C₁₈-Alky(meth)acrylat Isobutyl(meth)acrylat. Dementsprechend ist das erhaltene C₂-C₁₈-Alkyl(meth)acrylat beispielsweise Cyclohexyl(meth)acrylat, wenn als C₂-C₁₈-Alkohol Cyclohexanol umgesetzt wird.

Das in Schritt e) abgetrennte C₂-C₁₈-Alkyl(meth)acrylat enthält üblicherweise noch Reste des mindestens einen Alkylesters. Beispielsweise enthält das C₂-C₁₈-Alkyl(meth)acrylat im Bereich von 50 Gew.-ppm bis 8500 Gew.-ppm, bevorzugt im Bereich von 75 Gew.-ppm bis 4700 Gew.-ppm des mindestens einen Alkylesters, bezogen auf das Gesamtgewicht des C₂-C₁₈-Alkyl(meth)acrylats.

Gegenstand der vorliegenden Erfindung ist daher auch ein C₂-C₁₈-Alkyl(meth)acrylat, erhältlich nach dem erfindungsgemäßen Verfahren, wobei das C₂-C₁₈-Alkyl(meth)acrylat im Bereich von 50 ppm bis 10000 ppm des mindestens einen Alkylesters enthält, bezogen auf das Gesamtgewicht des C₂-C₁₈-Alkyl(meth)acrylats.

Ein weiterer Gegenstand der vorliegenden Erfindung ist ein Verfahren zur Isolierung von C₂-C₁₈-Alkyl(meth)acrylaten umfassend die folgenden Schritte c1) bis e):
c1) Mischen eines flüssigen ersten Stroms, der mindestens ein Alkyl(meth)acrylat und mindestens einen weiteren Alkylester enthält, wobei der flüssige erste Strom durch thermische Spaltung von mindestens einer Polymerzusammensetzung erhältlich ist, mit einem weiteren Strom, der mindestens ein weiteres Alkyl(meth)acrylat enthält, wobei der weitere Strom Teil eines C₂-C₁₈-Alkyl(meth)acrylat-Herstellverfahrens ist, unter Erhalt eines Mischstroms, der den flüssigen ersten Strom und den weiteren Strom umfasst,
d) Umsetzen des in Schritt c) erhaltenen Mischstroms mit einem C₂-C₁₈-Alkohol in Gegenwart eines Katalysators unter Erhalt eines Produktstroms, der das mindestens eine Alkyl(meth)acrylat, das mindestens eine weitere Alkyl(meth)acrylat, das C₂-C₁₈-Alkyl(meth)acrylat, den Katalysator, den mindestens einen Alkylester, den C₂-C₁₈-Alkohol und mindestens einen weiteren Alkohol enthält,
e) Abtrennung des C₂-C₁₈-Alkyl(meth)acrylats aus dem Produktstrom.

In Schritt c1) wird der flüssiger erster Strom mit dem weiteren Strom gemischt. Der flüssige erste Strom ist erhältlich durch thermische Spaltung von mindestens einer Polymerzusammensetzung. Für die Polymerzusammensetzung gelten die zuvor beschriebenen Ausführungen und Bevorzugungen.

Bevorzugt ist der flüssige erste Strom daher erhältlich durch die folgenden Schritte a) und b):
a) thermische Spaltung von mindestens einer Polymerzusammensetzung, die mindestens ein Poly(alkyl(meth)acrylat) enthält, unter Erhalt eines ersten Gasstroms, der mindestens ein Alkyl(meth)acrylat und mindestens einen weiteren Alkylester enthält,
b) Kondensation des in Schritt a) erhaltenen ersten Gasstroms unter Erhalt des flüssigen ersten Stroms, der das mindestens eine Alkyl(meth)acrylat und den mindestens einen weiteren Alkylester enthält.

Für die Schritte a) und b) gelten die zuvor beschriebenen Ausführungen und Bevorzugungen entsprechend.

Ebenso gelten für den flüssigen ersten Strom die zuvor beschriebenen Ausführungen und Bevorzugungen entsprechend. Für den Schritt c1) gelten die zuvor für Schritt c) beschriebenen Ausführungen und Bevorzugungen entsprechend.

Für die Schritte d) und e) gelten die zuvor beschriebenen Ausführungen entsprechend.

### Bezugszeichenliste

In den Figuren haben die Bezugszeichen folgende Bedeutung:
- 1: flüssiger erster Strom
- 2: weiterer Strom
- 3: Mischstrom
- 4: C₂-C₁₈-Alkohol
- 5: erste Destillationskolonne
- 6: Reaktor
- 7: zweiter Kopfstrom
- 8: zweiter Sumpfstrom
- 9: Niedrigsieder-Destillationskolonne
- 10: dritter Kopfstrom
- 11: dritter Sumpfstrom
- 12: Hochsieder-Destillationskolonne
- 13: vierter Kopfstrom
- 14: vierter Sumpfstrom
- 15: Katalysator
- 16: Produktstrom

### Figuren

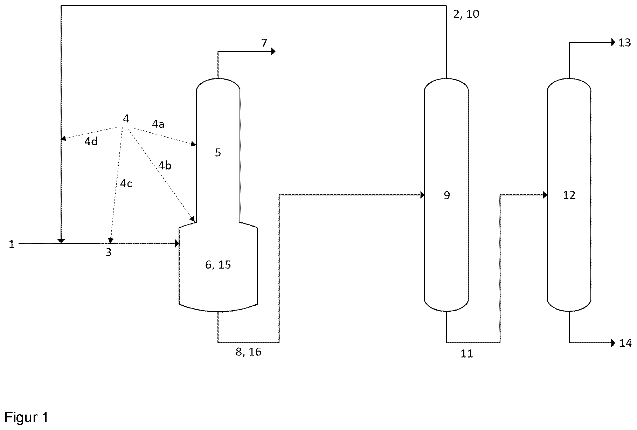

Figur 1 zeigt eine Ausführungsform der Schritte c) bis e) des erfindungsgemäßen Verfahrens.

Der flüssige erste Strom 1 wird mit dem weiteren Strom 2 gemischt unter Erhalt eines Mischstroms 3. In der gezeigten Ausführungsform werden die Schritte c) und d) des erfindungsgemäßen Verfahrens nacheinander durchgeführt. Als weiterer Strom 2 wird der dritte Kopfstrom 10 verwendet.

Der Mischstrom wird im Reaktor 6 mit dem C₂-C₁₈-Alkohol 4 umgesetzt. Der C₂-C₁₈-Alkohol 4 kann direkt in den Reaktor 6 gegeben werden (Zuleitung 4b). Zusätzlich oder alternativ ist es möglich, den C₂-C₁₈-Alkohol 4 in die erste Destillationskolonne 5 einzubringen (Zuleitung 4a). Zusätzlich oder alternativ ist es möglich, den C₂-C₁₈-Alkohol 4 mit dem Mischstrom 3 (Zuleitung 4c) und/oder dem weiteren Strom 2 zu mischen (Zuleitung 4d).

Im Reaktor 6 wird der Mischstrom 3 mit dem C₂-C₁₈-Alkohol 4 in Gegenwart eines Katalysators 15 umgesetzt unter Erhalt eines zweiten Kopfstroms 7, der über die erste Destillationskolonne 5 abgezogen wird, und eines zweiten Sumpfstroms 8, der den Produktstrom 16 umfasst. Der zweite Sumpfstrom 8 wird in die Niedrigsieder-Destillationskolonne 9 (zweite Destillationskolonne) überführt und dort destilliert unter Erhalt des dritten Kopfstroms 10 und des dritten Sumpfstroms 11. Der dritte Kopfstrom 10 wird in Schritt c) rückgeführt und mit dem flüssigen ersten Strom 1 gemischt. Der dritte Sumpfstrom 11 wird in die Hochsieder-Destillationskolonne 12 (dritte Destillationskolonne) überführt und dort destilliert unter Erhalt des vierten Kopfstroms 13, der das C₂-C₁₈-Alkyl(meth)acrylat enthält, und eines vierten Sumpfstroms 14. Der vierte Sumpfstrom 14 kann in Schritt d) rückgeführt werden (in Figur 1 nicht gezeigt).

### Beispiele

### Beispiele Depolymerisation

### Beispiel 1 - 5 - Depolymerisation in Abhängigkeit der Temperatur:

Jeweils ca. 50 mg PMMA Formmasse (Typ: Y7H) wurden bei unterschiedlichen Temperaturen gespalten und das aufgefangenen Kondensat per GC-MS untersucht. Die quantitative Bestimmung der Komponenten erfolgte durch Headspace GC-MS-Analyse. Die Ergebnisse sind in Tabelle 1 gezeigt.

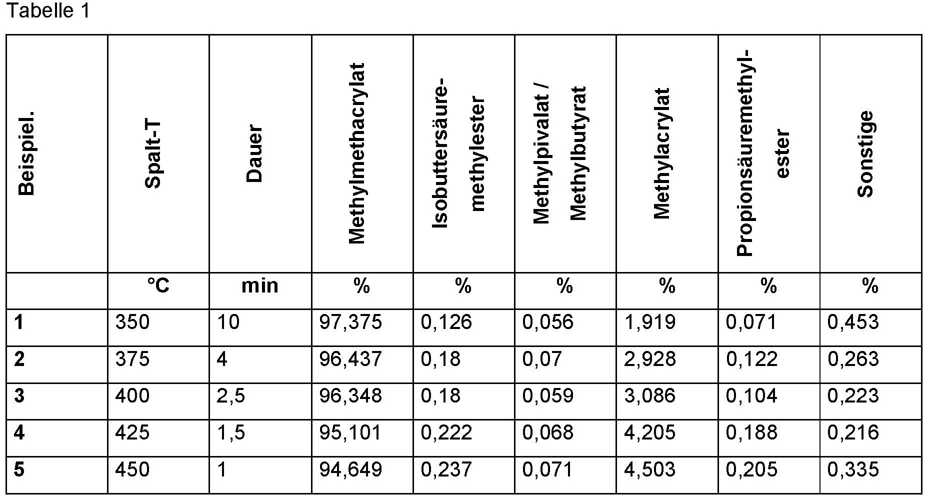

### Allg. Beschreibung Beispiel 6 - 13 kontinuierliche Depolymerisation

Reaktor: bestehend aus zwei Rohren (DN 100, aus Stahl 1.4571, je ca. 80 cm lang) mit aufgesetzten Schaugläsem (Schott-Glasbauteile, DN 100), die über ein 90° Winkelstück (DN 100, 1.4571) verbunden sind. Zur Temperaturerfassung mit Thermoelementen bestückt.

Beheizung: Schmelz- und Crack-Rohr mit je einer elektr. Heizmanschette (KJT, 220 V, 3000 W, 54 cm lang), Rohrbogen und Destillatübergang mit elektrischen Heizbändern. Heiztemperatur geregelt über Eurothermregler. Granulateintrag: Metallbehälter mit Kugelhahn und Stickstoffspülung (Eigenbau, ca. 5 I Fassungsvermögen) aufgesetzt auf einen Glaskühler (Schott, DN 100) mit eingesetztem Trichter (PE-Pulvertrichter mit Teflonrohr verlängert).

### Beschreibung:

Über einen mit Stickstoff gespülten Vorlagetrichter wird PMMA Granulat (Polymerzusammensetzung) über einen Trichter in den Vorwärmer des Reaktors eingetragen. Zur Erzeugung einer ausreichend fließfähigen Schmelze sind im Vorwärmer Temperaturen notwendig, bei denen eine beginnende Pyrolyse zu verzeichnen ist (geringfügig bei 250 °C, im zunehmenden Maße ab 300 °C). Bei zu niedrigen Heizmanteltemperaturen erfolgte ein nur unzureichender Energieeintrag (Wärmeaustauschfläche zu gering) und der Crackreaktor (temperiert auf 300-350 °C) wird nicht ausreichend mit Schmelze versorgt. Der Stickstoffstrom verhindert zudem eine Rückmischung der durch Depolymerisation entstandenen Gase und trägt diese in die Reaktionszone, in der das in der Schmelze enthaltene PMMA gespalten wird, weiter. Die Schmelze fließt durch Gravitation in die Reaktionszone weiter und teilt sich in nicht verdampfbaren Anteil und gasförmiges Polymerisat auf, letzteres wird kondensiert und gesammelt um ein für die Depolymerisation repräsentatives Kondensat zu erhalten. Je nach ursprünglicher Zusammensetzung und Reinheit des eingesetzten PMMA unterscheidet sich die Reinheit und Zusammensetzung des kondensierten flüssigen ersten Stroms ("Crack-MMA") signifikant. Die Verschiedenen Zusammensetzungen wurden mittels GC bestimmt und sind in Tabelle 2 gezeigt.

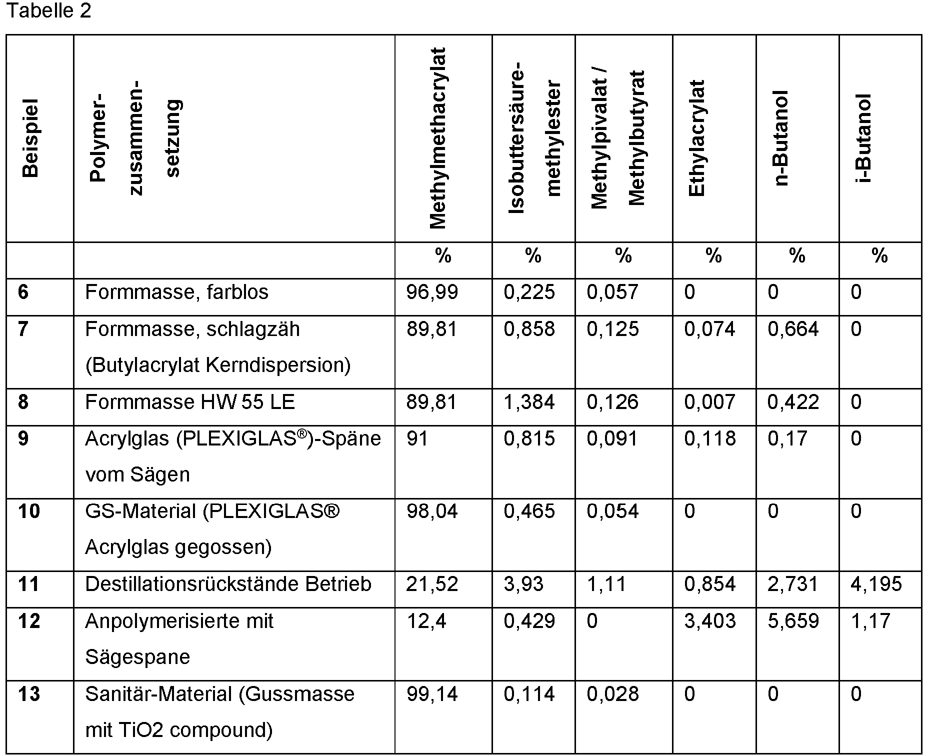

Deutlich ist zu erkennen, das bevorzugt nicht organisch modifizierte PMMA Rückstände zu verwenden sind, anorganische Verunreinigungen Bsp. 13 jedoch kaum Probleme bereiten.

### Beispiel 14 - 21: kontinuierliche Rektifikation von "Crack-MMA"

In einer Rektifikation wurde die Nieder- und Hochsiederabtrennung zur Herstellung von rein MMA aus dem flüssigen ersten Strom ("Crack-MMA") nachgestellt. Für die Versuche wurde eine Packungskolonne verwendet, die mit Drahtgewebepackungen der Firma Sulzer (Typ DX, DN 50; 2,3 m Packungshöhe) bestückt war. Die einzelnen Packungsbetten waren mit einem Flüssigkeitssammlersystem versehen, um eine Probennahme zu ermöglichen. Der Feed wurde vorgeheizt in die Mitte der Kolonne aufgegeben. Als Verdampfer wurde ein mit Wärmeträgeröl betriebener Robert-Verdampfer verwendet. Das Kopfprodukt wurde über ein Rücklaufteiler (Firma NGW) entnommen. Das Rücklaufverhältnis wurde über einen vorgegebenen Zeittakt des Steuergerätes eingestellt. Zur Kondensation der Dämpfe wurde eine Kombination aus Wasser- und Solekühler gewählt. Die Vorlauftemperatur des Wassers betrug etwa 9°C die der Sole etwa -12°C. Zur restlosen Kondensation der Dämpfe war eine mit Aceton/Trockeneis gefüllte Kältefalle nachgeschaltet. Das Sumpfprodukt lief frei in einen Vorlagekolben ab. Das Flüssigkeitsniveau des Verdampfers wurde über die Anordnung des Überlaufes bestimmt. Die Stabilisierung der Kolonne erfolgte durch eingeperlte Luft in den Kolonnensumpf und zugeführte Stabilisatorlösung am Kopf der Kolonne. Die Destillation wurde zweimal abgetrennt um zunächst eine Abtrennung von Leichtsiedern und Anschließend eine Entfernung von Hochsiedern zu erhalten, welche final das Reinprodukt als ersten Kopfstrom erhielt.

In mehreren Kampagnen wurde so Destillate gesammelt, deren Zusammensetzung in Tabelle 3 gezeigt ist:

**Tabelle 3**

| **Beispiel** | **14** | **15** | **16** | **17** | **18** | **19** | **20** | **21** |
|---|---|---|---|---|---|---|---|---|
| **MMA [GC-FI-%]** | 97,6 | 96,6 | 98,5 | 96,4 | 91,2 | 71,5 | 96,4 | 94,9 |
| **Methylpropionat [GC-FI-%]** | 0,1 | 0,2 | 0,15 | 0,1 | 0,2 | 0,2 | 0,2 | 0,1 |
| **Methylisobutyrat [GC-FI-%]** | 0,5 | 0,9 | 0,5 | 0,1 | 0,2 | 0,3 | 0,1 | 0,2 |
| **Methylpivalat [GC-FI-%]** | n.v. | n.v. | n.v. | 0,02 | 0,05 | 0,1 | 0,1 | n.v. |
| **Methylacrylat [GC-FI-%]** | 0,5 | 0,8 | 0,65 | 0,02 | 2,5 | 0,9 | 3,1 | 0,9 |
| **Schwefelkomponenten[ppm]** | 10 | 12 | 79 | n.b. | n.b. | n.b. | n.b. | n.b. |
| **(Di)carbonsäure-(di)methylester [GC-FI-%]** | 0,2 | 0 | 0 | 3,0 | 5,3 | 24,2 | 0 | 0 |
| **Oligomere und Hochsieder [GC-FI-%]** | 0 | 0 | 0 | 0 | 0 | 0 | 0 | 2,3 |

### Beschreibung der Herstellung von C₂-C₁₈-Alkyl(meth)acrylat:

Als Reaktor 6 wurde ein mit Wasserdampf beheizter Edelstahl-Reaktionskessel mit einem maximalen Füllvolumen von 15 I verwendet. Der Reaktor 6 war über eine Brüdenleitung mit einer oberhalb montierten Azeotrop-Destillationskolonne 5 verbunden. Bei der Azeotrop-Destillationskolonne 6 (Kopfdruck = 1 barabs) handelte es sich um eine mit H = 2 m Sulzer CY Metalldrahtgewebepackungen bestückte Technikums-Glaskolonne mit einem Durchmesser von D = 0,1 m. In der Kolonnenmitte (H = 1 m) befand sich der Zulauf 4a für den C₂-C₁₈-Alkohol 4. Der zweite Sumpfstrom 8 wurde einer Niedrigsieder-Destillationskolonne 9 kontinuierlich zugeführt. Bei der Niedrigsieder-Destillationskolonne 9 handelte es sich um eine mit H = 3,8 m Sulzer CY Metalldrahtgewebepackungen bestückte Technikums-Vakuumglaskolonne (Kopfdruck = 120 mbarabs) mit einem Durchmesser von D = 0,1 m. Der Zulauf befand sich auf H = 2 m. Die Sumpfbeheizung erfolgte mit Wasserdampf. Der kondensierte dritte Kopfstrom 2 wurde kontinuierlich zum Reaktor 6 zurückgeführt. Statt eines Fallfilmverdampfers kam bei der kontinuierlichen Aufarbeitung des dritten Sumpfstroms 11 der Niedrigsieder-Destillationskolonne 9 ein mit Thermalöl beheizter Glasdünnschichtverdampfer mit einer Verdampferfläche von A = 0,1 m² zum Einsatz. Die Brüden dieses Glasdünnschichtverdampfers wurden kontinuierlich in eine oberhalb montierte Hochsieder-Destillationskolonne 12 geleitet. Es handelte sich dabei um eine mit H = 0,5 m Sulzer EX Metalldrahtgewebepackungen bestückte Technikums-Vakuumglaskolonne (Kopfdruck = 120 mbarabs) mit einem Durchmesser von D = 0,05 m. Der vierte Sumpfstrom 14 wurde einem zweiten, kleineren, ebenfalls mit Thermalöl beheizten Glasdünnschichtverdampfer (Kopfdruck = 120 mbarabs) mit einer Verdampferfläche von A =0,02 m² kontinuierlich zugeführt. Die Brüden dieses zweiten Glasdünnschichtverdampfers wurden auskondensiert und vereinigt mit dem Reaktorabstrom der Niedrigsieder-Destillationskolonne 9 kontinuierlich zugeführt. Der Sumpfabstrom wurde kontinuierlich aus dem Prozess ausgeschleust. Die Dosierung der Edukte (Methylmethacrlat, MMA, als flüssiger erster Strom 1 und C₂-C₁₈-Alkohol 4) erfolgte kontinuierlich mittels Kolben-Dosierpumpen, wobei der Katalysator 15 (Tetraalkyltitanat) im (per Spezifikation wasserfreien) MMA-Feed (Mischstrom 3) gelöst zudosiert wurde. Der MMA/Katalysator-Feed wurde dem Reaktor direkt zugeführt, der C₂-C₁₈-Alkohol-Feed wurde (auf Kolonneninnentemperatur) vorgewärmt auf die Mitte der Azeotrop-Destillationskolonne 5 aufgegeben. Die kontinuierliche Zugabe von 50 -100 g/h Stabilisatorlösung (0,2 Gew.-% Hydrochinonmonomethylether in MMA bzw. Produktester) erfolgte mit Hilfe von Schlauchpumpen in den Rücklaufstrom der Destillationskolonnen. Die kontinuierliche Förderung der Stoffströme zwischen den Anlagenteilen erfolgte entweder mit Hilfe von Kolben-Dosierpumpen oder durch die Saugwirkung des Vakuums. Zwischenbehälter (Puffervolumina) wurden, außer bei den Kolonnenrückläufen, soweit als möglich vermieden. Die Zusammensetzung der Stoffströme wurde mit Hilfe eines Gaschromatographen bestimmt.

### Beispiel 22: Herstellung von n-Butylmethacrylat

Zur kontinuierlichen Herstellung von n-Butylmethacrylat (n-BuMA) wurden dem Reaktionskessel 2,62 kg/h MMA-Feed aus einer Depolymerisation von PMMA (flüssiger erster Strom, erster Kopfstrom), 2,5 g/h Tetra-n-Butyltitanat (Ti(n-OBu)₄) (Katalysator) und 1,78 kg/h n-BuOH-Feed (C₂-C₁₈-Alkohol) zudosiert. Weiterhin floß dem Reaktor 1,84 kg/h des Kreislaufrückstrom vom Kopf der Niedrigsieder-Destillationskolonne (dritter Kopfstrom) kontinuierlich zu. Das molare MMA : n-BuOH-Verhältnis im Reaktorzulauf betrug 1,1 : 1. Bei einer Reaktorverweilzeit von 2,67 h und einem Azeotropabzug (zweiter Kopfstrom) von 1,0 kg/h stellte sich eine Reaktortemperatur von 115 °C ein. Der resultierende Reaktorabstrom (zweiter Sumpfstrom) betrug 5,6 kg/h. Aufgrund der Rückführung niedrigsiedender Komponenten wurde im Sumpfaustrag der Niedrigsieder-Destillationskolonne (dritter Sumpfstorm) ein Rohester erhalten (4 kg/h), der bereits > 98,4 Gew.-% n-BuMA sowie den gesamten Katalysator und Stabilisator enthielt. Die Ausbeute an n-BuOH bezogen auf den gesamten Prozess betrug folglich nahezu 100 %. Die Ausbeute an MMA bezogen auf den gesamten Prozess betrug abzüglich des zuvor einkalkulierten MMA-Verlustes über das MMA/MeOH-Azeotrop ebenfalls nahezu 100 %. Bei einem Eindampfverhältnis (Verhältnis von Brüden- zu Feedstrom) im ersten, größeren Dünnschichtverdampfer von ca. 90 % werden am Kopf der Hochsieder-Destillationskolonne (vierter Kopfstrom) schließlich 3,4 kg/h Rein-n-BuMA gewonnen. Die Gesamtausschleusung (vierter Sumpfstrom) des Prozesses (Katalysator, Stabilisator, hochsiedende Nebenprodukte, n-BuMA) liegt bei 20 g/h.

Die Zusammensetzung aller oben genannter Prozessströme nach Erreichen eines stationären Zustands der Anlage ist in den folgenden Tabellen 4 bis 6 gezeigt, welche unterschiedliche reine Kopfströme aus Beispielen 14 bis 16 verwendeten.

**Tabelle 4 Verwendung der Fraktion aus Bsp. 14**

| | **Gew.-%** | | | | | | | | | | |
|---|---|---|---|---|---|---|---|---|---|---|---|
| | Alkyl(meth)- acrylat | C₂-C₁₈- Alkohol | weiterer Alkohol | C₂-C₁₈-Alkyl(meth)acrylat | | | weiterer Alkylester | | | | |
| | MMA | n- Butanol | Methanol | n-Butyl- methacrylat | Methyl- acrylat | n-Butyl-acrylat | Methyl- propionat | n-Butyl- propionat | Methyl- isobutyrat | n-Butylisobutyrat | Wasser |
| **3. Kopfstrom** | 57,69 | 36,78 | 2,09 | 2,10 | 0,15 | 0,38 | 0,02 | 0,05 | 0,29 | 0,11 | 0 |
| **C₂-C₁₈-Alkohol Feed** | 0,00 | 99,91 | 0,00 | 0,00 | 0,00 | 0,00 | 0,00 | 0,00 | 0,00 | 0,00 | 0,02 |
| **1. Kopfstrom** | 98,89 | 0,00 | 0,00 | 0,00 | 0,51 | 0,00 | 0,10 | 0,00 | 0,51 | 0,00 | 0,00 |
| **2. Sumpfstrom** | 18,89 | 12,04 | 0,69 | 67,43 | 0,05 | 0,19 | 0,01 | 0,03 | 0,10 | 0,34 | 0 |
| **4. Sumpfstrom** | 0,00 | 0,00 | 0,00 | 85,77 | 0,00 | 0,02 | 0,00 | 0,00 | 0,00 | 0,22 | 0,00 |
| **% des feed** | | | | 0,47 | | 0,02 | | 0,02 | | 0,23 | |
| **2. Kopfstrom** | 20,66 | 0,00 | 77,74 | 0,00 | 1,11 | 0,00 | 0,22 | 0,00 | 0,20 | 0,00 | 0,04 |
| **% des feed** | 7,90 | | | | 83,07 | | 83,20 | | 14,86 | | |
| **3. Sumpfstrom** | 0,00 | 0,00 | 0,00 | 98,42 | 0,00 | 0,09 | 0,00 | 0,02 | 0,00 | 0,44 | 0 |
| **4. Kopfstrom** | 0,00 | 0,00 | 0,00 | 99,41 | 0,00 | 0,10 | 0,00 | 0,02 | 0,00 | 0,47 | 0 |
| **3. Kopfstrom** | | | | 92,15 | | 16,90 | | 16,77 | | 84,91 | |

**Tabelle 5 Verwendung der Fraktion aus Bsp. 15**

| | **Gew.-%** | | | | | | | | | | |
|---|---|---|---|---|---|---|---|---|---|---|---|
| | Alkyl(meth)- acrylat | C₂-C₁₈- Alkohol | weiterer Alkohol | C₂-C₁₈-Alkyl(meth)acrylat | | | weiterer Alkylester | | | | |
| | MMA | n- Butanol | Methanol | n-Butyl- methacrylat | Methyl- acrylat | n-Butyl-acrylat | Methyl- propionat | n-Butyl- propionat | Methyl- isobutyrat | n-Butylisobutyrat | Wasser |
| **3. Kopfstrom** | 57,56 | 36,18 | 2,07 | 2,10 | 0,25 | 0,62 | 0,05 | 0,11 | 0,53 | 0,20 | 57,56 |
| **C₂-C₁₈-Alkohol Feed** | 0,00 | 99,91 | 0,00 | 0,00 | 0,00 | 0,00 | 0,00 | 0,00 | 0,00 | 0,00 | 0,00 |
| **1. Kopfstrom** | 98,07 | 0,00 | 0,00 | 0,00 | 0,81 | 0,00 | 0,20 | 0,00 | 0,91 | 0,00 | 98,07 |
| **2. Sumpfstrom** | 18,90 | 11,87 | 0,68 | 67,06 | 0,08 | 0,30 | 0,02 | 0,06 | 0,17 | 0,61 | 18,90 |
| **4. Sumpfstrom** | 0,00 | 0,00 | 0,00 | 85,54 | 0,00 | 0,03 | 0,00 | 0,01 | 0,00 | 0,39 | 0,00 |
| **% des feed** | | | | 0,47 | | 0,02 | | 0,02 | | 0,24 | |
| **2. Kopfstrom** | 20,18 | 0,00 | 77,17 | 0,00 | 1,79 | 0,00 | 0,45 | 0,00 | 0,36 | 0,00 | 20,18 |
| **% des feed** | 7,82 | | | | 83,54 | | 83,81 | | 14,88 | | 7,82 |
| **3. Sumpfstrom** | 0,00 | 0,00 | 0,00 | 98,13 | 0,00 | 0,14 | 0,00 | 0,03 | 0,00 | 0,80 | 0,00 |
| **4. Kopfstrom** | 0,00 | 0,00 | 0,00 | 98,96 | 0,00 | 0,15 | 0,00 | 0,04 | 0,00 | 0,84 | 0,00 |
| **% des feed** | | | | 92,24 | | 16,43 | | 16,15 | | 84,88 | |

**Tabelle 6 Verwendung der Fraktion aus Bsp. 16**

| | **Gew.-%** | | | | | | | | | | |
|---|---|---|---|---|---|---|---|---|---|---|---|
| | Alkyl(meth)- acrylat | C₂-C₁₈- Alkohol | weiterer Alkohol | C₂-C₁₈-Alkyl(meth)acrylat | | | weiterer Alkylester | | | | |
| | MMA | n- Butanol | Methanol | n-Butyl- methacrylat | Methyl- acrylat | n-Butyl-acrylat | Methyl- propionat | n-Butyl- propionat | Methyl- isobutyrat | n-Butylisobutyrat | Wasser |
| **3. Kopfstrom** | 57,55 | 36,73 | 2,08 | 2,10 | 0,20 | 0,49 | 0,04 | 0,08 | 0,29 | 0,11 | 0 |
| **C₂-C₁₈-Alkohol Feed** | 0,00 | 99,91 | 0,00 | 0,00 | 0,00 | 0,00 | 0,00 | 0,00 | 0,00 | 0,00 | 0,02 |
| **1. Kopfstrom** | 98,69 | 0,00 | 0,00 | 0,00 | 0,65 | 0,00 | 0,15 | 0,00 | 0,50 | 0,00 | 0,00 |
| **2. Sumpfstrom** | 18,87 | 12,03 | 0,69 | 67,38 | 0,06 | 0,24 | 0,01 | 0,04 | 0,09 | 0,33 | 0 |
| **4. Sumpfstrom** | 0,00 | 0,00 | 0,00 | 85,73 | 0,00 | 0,03 | 0,00 | 0,01 | 0,00 | 0,21 | 0,00 |
| **% des feed** | | | | 0,47 | | 0,02 | | 0,02 | | 0,23 | |
| **2. Kopfstrom** | 20,73 | 0,00 | 77,23 | 0,00 | 1,42 | 0,00 | 0,33 | 0,00 | 0,20 | 0,00 | 0,06 |
| **% des feed** | 7,97 | | | | 83,19 | | 83,33 | | 15,07 | | |
| **3. Sumpfstrom** | 0,00 | 0,00 | 0,00 | 98,41 | 0,00 | 0,11 | 0,00 | 0,03 | 0,00 | 0,44 | 0 |
| **4. Kopfstrom** | 0,00 | 0,00 | 0,00 | 99,38 | 0,00 | 0,12 | 0,00 | 0,03 | 0,00 | 0,46 | 0 |
| **% des feed** | | | | 92,08 | | 16,78 | | 16,64 | | 84,69 | |

## Patentansprüche

1. Verfahren zur Isolierung von C₂-C₁₈-Alkyl(meth)acrylaten umfassend die folgenden Schritte a) bis e):
a) thermische Spaltung von mindestens einer Polymerzusammensetzung, die mindestens ein Poly(alkyl(meth)acrylat) enthält, unter Erhalt eines ersten Gasstroms, der mindestens ein Alkyl(meth)acrylat und mindestens einen weiteren Alkylester enthält,
b) Kondensation des in Schritt a) erhaltenen ersten Gasstroms unter Erhalt eines flüssigen ersten Stroms, der das mindestens eine Alkyl(meth)acrylat und den mindestens einen weiteren Alkylester enthält,
c) Mischen des in Schritt b) erhaltenen flüssigen ersten Stroms mit einem weiteren Strom, der mindestens ein weiteres Alkyl(meth)acrylat enthält, wobei der weitere Strom Teil eines C₂-C₁₈-Alkyl(meth)acrylat-Herstellverfahrens ist, unter Erhalt eines Mischstroms, der den flüssigen ersten Strom und den weiteren Strom umfasst,
d) Umsetzen des in Schritt c) erhaltenen Mischstroms mit einem C₂-C₁₈-Alkohol in Gegenwart eines Katalysators unter Erhalt eines Produktstroms, der das mindestens eine Alkyl(meth)acrylat, das mindestens eine weitere Alkyl(meth)acrylat, das C₂-C₁₈-Alkyl(meth)acrylat, den Katalysator, den mindestens einen Alkylester, den C₂-C₁₈-Alkohol und mindestens einen weiteren Alkohol enthält,
e) Abtrennung des C₂-C₁₈-Alkyl(meth)acrylats aus dem Produktstrom.

2. Verfahren gemäß Anspruch 1, **dadurch gekennzeichnet, dass** die Polymerzusammensetzung in Schritt a) zumindest eine weitere Komponente, ausgewählt aus der Gruppe bestehend aus von Poly(alkyl(meth)acrylat) verschiedenen Polymeren, Pigmenten, Farbstoffen, Füllstoffen, Hilfsstoffen, Reglern, Initiatoren, Schlagzähmachern, Trennmitteln und UV-Additiven, enthält.

3. Verfahren gemäß Anspruch 1 oder 2, **dadurch gekennzeichnet, dass** das in dem ersten Gasstrom in Schritt a) enthaltene mindestens eine Alkyl(meth)acrylat ausgewählt ist aus der Gruppe bestehend aus C₁- bis C₄-Alkyl(meth)acrylaten.

4. Verfahren gemäß einem der Ansprüche 1 bis 3, **dadurch gekennzeichnet, dass** der mindestens eine weitere Alkylester ausgewählt ist aus der Gruppe bestehend aus Methylpropionat, Methylisobutyrat, Methylpivalat, Methyl-3-methoxyisobutyrat und Dicarbonsäurediestern.

5. Verfahren gemäß einem der Ansprüche 1 bis 4, **dadurch gekennzeichnet, dass** der erste Gasstrom mindestens eine weitere Komponente, ausgewählt aus der Gruppe bestehend aus Styrol, (Meth)acrylsäure, schwefelhaltigen Verbindungen, Oligomeren und Dimeren, enthält.

6. Verfahren gemäß einem der Ansprüche 1 bis 5, **dadurch gekennzeichnet, dass** der erste Gasstrom nach Schritt a) und vor Schritt b) destilliert wird unter Erhalt eines ersten Kopfstroms, der das mindestens eine Alkyl(meth)acrylat und den mindestens einen weiteren Alkylester enthält, und eines ersten Sumpfstroms, der mindestens eine von dem mindestens einen Alkyl(meth)acrylat und dem mindestens einen weiteren Alkylester verschiedene Komponente enthält, wobei der erste Kopfstrom in Schritt b) kondensiert wird.

7. Verfahren gemäß einem der Ansprüche 1 bis 6, **dadurch gekennzeichnet, dass** der C₂-C₁₈-Alkohol in Schritt d) ein Polyol umfasst.

8. Verfahren gemäß einem der Ansprüche 1 bis 7, **dadurch gekennzeichnet, dass** der Katalysator ausgewählt ist aus der Gruppe bestehend aus Metallsalzen und organischen Verbindungen, jeweils von Metallen ausgewählt aus der Gruppe bestehend aus Zinn, Titan, Zirkon, Alkalimetallen und Erdalkalimetallen.

9. Verfahren gemäß einem der Ansprüche 1 bis 8, **dadurch gekennzeichnet, dass** die Schritte c) und d) zeitgleich durchgeführt werden.

10. Verfahren gemäß einem der Ansprüche 1 bis 9, **dadurch gekennzeichnet, dass** bei der Umsetzung in Schritt d) ein zweiter Kopfstrom, der das mindestens eine Alkyl(meth)acrylat, das mindestens eine weitere Alkyl(meth)acrylat, den mindestens einen Alkylester und den mindestens einen weiteren Alkohol enthält, und ein zweiter Sumpfstrom, der den Produktstrom umfasst, erhalten wird.

11. Verfahren gemäß Anspruch 10, **dadurch gekennzeichnet, dass** der erhaltene zweite Kopfstrom in Schritt c) und/oder Schritt d) rückgeführt wird und/oder einem Verfahren zur Herstellung eines C₁-C₂-Alkyl(meth)acrylats zugeführt wird.

12. Verfahren gemäß einem der Ansprüche 1 bis 11, **dadurch gekennzeichnet, dass** Schritt e) den folgenden Schritt e1) umfasst:
e1) Destillation des Produktstroms unter Erhalt eines dritten Kopfstroms, der das mindestens eine Alkyl(meth)acrylat, das mindestens eine weitere Alkyl(meth)acrylat, den mindestens einen Alkylester, den C₂-C₁₈-Alkohol und den mindestens einen weiteren Alkohol enthält, und eines dritten Sumpfstroms, der das C₂-C₁₈-Alkyl(meth)acrylat, Reste des mindestens einen Alkylesters und den Katalysator enthält.

13. Verfahren gemäß Anspruch 12, **dadurch gekennzeichnet, dass** der dritte Kopfstrom in Schritt c) und/oder Schritt d) rückgeführt wird und/oder einem Verfahren zur Herstellung eines C₁-C₂-Alkyl(meth)acrylats zugeführt wird.

14. Verfahren gemäß Anspruch 12 oder 13, **dadurch gekennzeichnet, dass** im Anschluss an Schritt e1) der folgende Schritt e2) durchgeführt wird:
e2) Destillation des in Schritt e1) erhaltenen dritten Sumpfstroms unter Erhalt eines vierten Kopfstroms, der das C₂-C₁₈-Alkyl(meth)acrylat enthält, und eines vierten Sumpfstroms, der die Reste des mindestens einen Alkylesters und den Katalysator enthält.

15. Verfahren gemäß Anspruch 14, **dadurch gekennzeichnet, dass** der vierte Sumpfstrom in Schritt d) rückgeführt wird.

16. C₂-C₁₈-Alkyl(meth)acrylat erhältlich nach einem Verfahren gemäß einem der Ansprüche 1 bis 15, **dadurch gekennzeichnet, dass** das C₂-C₁₈-Alkyl(meth)acrylat im Bereich von 50 ppm bis 10000 ppm des mindestens einen Alkylesters enthält, bezogen auf das Gesamtgewicht des C₂-C₁₈-Alkyl(meth)acrylats.
